# EUROPEAN PATENT APPLICATION

(11) **EP 4 445 906 A1**
(43) Date of publication of application: **16.10.2024**
(21) Application number: 22903573.8
(22) Date of filing: 08.12.2022
(51) Int. Cl.: A61K 38/10, A61K 39/00, A61K 39/215, C07K 7/08, C07K 16/10

(54) **ANTIGENIC POLYPEPTIDE AND USE THEREOF**

(30) Priority: 08.12.2021 CN 202111491373
(71) Applicant: Anda Biology Medicine Development (Shenzhen) Co., Ltd., Shenzhen, Guangdong 518108 (CN)
(72) Inventor: HU, Landian, Shenzhen, Guangdong 518108 (CN)
(74) Representative: Maiwald GmbH
(86) International application number: PCT/CN2022/137554
(87) International publication number: WO 2023/104154

(57) **Abstract**

Provided are an isolated polypeptide or an antigenic fragment thereof, an antibody binding to the isolated polypeptide or the antigenic fragment thereof, a composition including the isolated polypeptide or the antigenic fragment thereof, and an array including the isolated polypeptide or the antigenic fragment thereof. Further provided a method for detecting the presence of an antibody against SARS-CoV-2 in a subject, a method for the prognosis of the subject infected with SARS-CoV-2, a method for inducing an immune response against SARS-CoV-2 in a subject, and a method for preventing and/or treating a disease caused by SARS-CoV-2 infection in a subject.

## Description

### TECHNICAL FIELD

The present disclosure relates to an isolated polypeptide, an antigenic fragment thereof, and a use of the isolated polypeptide for inducing an immune response and diagnosing and/or treating a disease.

### BACKGROUND

Coronavirus disease 2019 (COVID-19) has now become a global pandemic, and its pathogen is SARS-CoV-2 (Zhou, P., et al., A pneumonia outbreak associated with a new coronavirus of probable bat origin. Nature, 2020. 579 (7798): p. 270-273). As of February 24, 2021, a total of 111,762,993 diagnosed cases have been reported globally, and there have been 101,778 accumulative diagnosed cases in China. In order to control this pandemic, one of the basic tasks is fast executed, reliable and affordable diagnosis. So far, detection of viral infections has always relied on extensively screening isolated individuals with nucleic acid detection. Sensitivity of nucleic acid detection depends largely on a course and a type of clinical COVID-19 syndrome, a collection site, and transportation and storage of a specimen. It is reported that a false negative rate of nucleic acid detection is about 30% in patients with COVID-19 (Ai, T., et al., Correlation of Chest CT and RT-PCR Testing for Coronavirus Disease 2019 (COVID-19) in China: A Report of 1014 Cases. Radiology, 2020. 296 (2): p. E32-E40). A recent survey on positive rate of nucleic acid detection shows that COVID-19 cases in different countries and regions, especially asymptomatic cases, are greatly underestimated. Therefore, due to limited sensitivity of nucleic acid detection, some asymptomatic infection cases may be ignored.

Use of serum antibody detection with more convenient and simple detection and interpretation can effectively improve reliability of a result, serving as double insurances for diagnosis. If serum samples of the patients with COVID-19 are collected within 19 d after the onset of symptoms, the sensitivity of an IgG antibody response to SARS-CoV-2 may be close to 100% (Long, Q.X., et al., Antibody responses to SARS-CoV-2 in patients with COVID-19. Nat Med, 2020. 26 (6): p. 845-848). In addition, antibodies that have a strong neutralization reaction against viral proteins can effectively resist the viruses. Antibody responses in sera of convalescent play an important role in patients' recovery, and these antibodies have a same therapeutic effect for other infected patients (Jiang, L., et al., Potent neutralization of MERS-CoV by human neutralizing monoclonal antibodies to the viral spike glycoprotein. Sci Transl Med, 2014. 6 (234): p. 234ra59). Therefore, whether it is for diagnostic detection, screening of therapeutic antibodies or for the preparation of vaccines, it is necessary to fully understand antigenicity of various proteins and various regions of the SARS-CoV-2, as well as changes in the patient's antibody responses to various proteins with disease progression.

Sequence analysis on the viruses shows that SARS-CoV-2 is similar to bat SARS coronavirus and SARS virus, belonging to a genus of β-coronavirus, and is most closely related to Bat-CoV RaTG13 virus (Zhou, P., et al., the same as above). Among them, a genome of the SARS-CoV-2 is about 80% similar to that of the SARS coronavirus in sequence. By comparing with other known protein sequences of coronaviruses, it is predicted that the SARS-CoV-2 can encode 29 proteins: 4 structural proteins, 9 accessory proteins and 16 non-structural proteins (Nsp) (Wu, A., et al., Genome Composition and Divergence of the Novel Coronavirus (2019-nCoV) Originating in China. Cell Host Microbe, 2020. 27 (3): p. 325-328). The structural proteins include a membrane protein (M protein), a nucleocapsid protein (N protein), a spike protein (S protein), and an envelope protein (E protein). These proteins are exposed to a surface of the virus, while the non-structural proteins wrap an internal of the virus. At present, it has been known from functional researches that among internal proteins, Orf6 and Orf9b inhibit interferon. Nsp1 inhibits an antiviral response, while Nsp15 and Orf3a activate the interferon respectively. NLRP3 and Orf7a are viral proteins responsible for inducing cell death.

There are evidences that the novel coronavirus and the SARS coronavirus use a same mechanism to enter host cells. That is, the viruses bind to the host cells through high affinity between a receptor binding domain (RBD) of the S protein and an angiotensin-converting enzyme 2 (ACE2) gene of a host (Li, W., et al., Angiotensin-converting enzyme 2 is a functional receptor for the SARS coronavirus. Nature, 2003. 426 (6965): p. 450-4).

Due to an interaction between the S protein and the host, the S protein, especially the RBD region, is widely used as a target for an antibody response in serum antibody detection at present (Long, Q.X., et al., Antibody responses to SARS-CoV-2 in patients with COVID-19. Nat Med, 2020. 26 (6): p. 845-848). Therapeutic antibodies and vaccines for treating COVID-19 are also directed against the S protein, especially the RBD region (Cao, Y., et al., Potent Neutralizing Antibodies against SARS-CoV-2 Identified by High-Throughput Single-Cell Sequencing of Convalescent Patients' B Cells. Cell, 2020. 182 (1): p. 73-84.e16). A key reagent for serological detection based on the S protein is a recombinant S protein. However, the S protein is difficultly produced with a high cost (Petherick, A., Developing antibody tests for SARS-CoV-2. Lancet, 2020. 395 (10230): p. 1101-1102). In addition, although the recombinant S protein is used, inconsistency between different manufacturers or even batches may lead to differences in test results (Lisboa, B.M., et al., Diagnostic accuracy of serological tests for covid-19: systematic review and meta-analysis. BMJ, 2020. 370: p. m2516). Moreover, cross-reactions caused by infection with other human coronaviruses may also lead to false positive results, especially for four common coronaviruses responsible for cold (HCoV-OC43, HKU1, NL63, and 229E) that are prevalent in the population (Petherick, A., the same as above). In order to develop a highly specific serum antibody test, it is necessary to identify a more specific region with strong antigenicity and a portion having lower homology with related coronaviruses.

### SUMMARY

In a first aspect, the present disclosure provides an isolated polypeptide or an antigenic fragment thereof, wherein the polypeptide has an amino acid sequence selected from a group consisting of the following amino acid sequences or amino acid sequences obtained after a substitution, a deletion, an insertion and/or an addition of one or more amino acid residues has been made in the following amino acid sequences:
SFKWDLTAFGLVAEW (SEQ ID NO: 1);
LGLAAIMQLFFSYFA (SEQ ID NO: 2);
VLSTFISAARQGFVD (SEQ ID NO: 3);
SFCYMHHMELPTGVH (SEQ ID NO: 4);
MQTMLFTMLRKLDND (SEQ ID NO: 5);
TCTERLKLFAAETLK (SEQ ID NO: 6);
KGVITHDVSSAINRP (SEQ ID NO: 7);
DQFKHLIPLMYKGLP (SEQ ID NO: 8);
SSVLHSTQDLFLPFF (SEQ ID NO: 9);
SKRSFIEDLLFNKVT (SEQ ID NO: 10);
YELQTPFEIKLAKKF (SEQ ID NO: 11);
EKYCALAPNMMVTNN (SEQ ID NO: 12);
ATYKPNTWCIRCLWS (SEQ ID NO: 13);
LKTLVATAEAELAKN (SEQ ID NO: 14);
MYLKLRSDVLLPLTQ (SEQ ID NO: 15);
YEDLLIRKSNHNFLV (SEQ ID NO: 16);
KVDTANPKTPK (SEQ ID NO: 17);
LGSALLEDEFTPFDV (SEQ ID NO: 18);
SEWLKKLKKSLNVA (SEQ ID NO: 19);
LEKMADQAMTQMYKQ (SEQ ID NO: 20);
VPLNIIPLTTAAKLM (SEQ ID NO: 21);
LNRGMVLGSLAATVR (SEQ ID NO: 22);
FCAFAVDAAKAYKDY (SEQ ID NO: 23);
FFKFRIDGDMVPHIS (SEQ ID NO: 24);
NLDKSAGFPFNKWGK (SEQ ID NO: 25);
VVYRGTTTYKLNVGD (SEQ ID NO: 26);
AKHYVYIGDPAQLPA (SEQ ID NO: 27);
PAEIVDTVSALVYDN (SEQ ID NO: 28);
KDKSAQCFKMFYKGV (SEQ ID NO: 29);
GFDYVYNPFMIDVQQ (SEQ ID NO: 30);
LIGDCATVHTANKWD (SEQ ID NO: 31);
TAFVTNVNASSSEAF (SEQ ID NO: 32);
LSSYSLFDMSKFPLK (SEQ ID NO: 33);
IDGYFKIYSKHTPIN (SEQ ID NO: 34);
ALQIPFAMQMAYRFN (SEQ ID NO: 35);
GGDAALALLLLDRL (SEQ ID NO: 36);
AALALLLLDRLNQLE (SEQ ID NO: 37);
GMEVTPSGTWLTYTG (SEQ ID NO: 38); and
IDAYKTFPPTEPKKD (SEQ ID NO: 39).

In a second aspect, the present disclosure provides an isolated polypeptide or an antigenic fragment thereof, wherein the polypeptide has an amino acid sequence selected from a group consisting of the following amino acid sequences or amino acid sequences obtained after a substitution, a deletion, an insertion and/or an addition of one or more amino acid residues has been made in the following amino acid sequences:
SFKWDLTAFGLVAEW (SEQ ID NO: 1);
LGLAAIMQLFFSYFA (SEQ ID NO: 2);
VLSTFISAARQGFVD (SEQ ID NO: 3);
SFCYMHHMELPTGVH (SEQ ID NO: 4);
MQTMLFTMLRKLDND (SEQ ID NO: 5);
TCTERLKLFAAETLK (SEQ ID NO: 6);
KGVITHDVSSAINRP (SEQ ID NO: 7);
DQFKHLIPLMYKGLP (SEQ ID NO: 8);
SSVLHSTQDLFLPFF (SEQ ID NO: 9);
SKRSFIEDLLFNKVT (SEQ ID NO: 10);
YELQTPFEIKLAKKF (SEQ ID NO: 11);
EKYCALAPNMMVTNN (SEQ ID NO: 12);
ATYKPNTWCIRCLWS (SEQ ID NO: 13);
LKTLVATAEAELAKN (SEQ ID NO: 14);
MYLKLRSDVLLPLTQ (SEQ ID NO: 15);
YEDLLIRKSNHNFLV (SEQ ID NO: 16);
KVDTANPKTPK (SEQ ID NO: 17);
LGSALLEDEFTPFDV (SEQ ID NO: 18);
SEWLKKLKKSLNVA (SEQ ID NO: 19);
LEKMADQAMTQMYKQ (SEQ ID NO: 20);
VPLNIIPLTTAAKLM (SEQ ID NO: 21);
LNRGMVLGSLAATVR (SEQ ID NO: 22);
FCAFAVDAAKAYKDY (SEQ ID NO: 23);
FFKFRIDGDMVPHIS (SEQ ID NO: 24);
NLDKSAGFPFNKWGK (SEQ ID NO: 25);
VVYRGTTTYKLNVGD (SEQ ID NO: 26);
AKHYVYIGDPAQLPA (SEQ ID NO: 27);
PAEIVDTVSALVYDN (SEQ ID NO: 28);
KDKSAQCFKMFYKGV (SEQ ID NO: 29);
GFDYVYNPFMIDVQQ (SEQ ID NO: 30);
LIGDCATVHTANKWD (SEQ ID NO: 31);
TAFVTNVNASSSEAF (SEQ ID NO: 32);
LSSYSLFDMSKFPLK (SEQ ID NO: 33);
IDGYFKIYSKHTPIN (SEQ ID NO: 34); and
ALQIPFAMQMAYRFN (SEQ ID NO: 35).

In some embodiments in the first aspect and the second aspect of the present disclosure, the antigenic fragment is at least 5 amino acids, at least 6 amino acids, at least 7 amino acids, at least 8 amino acids, at least 9 amino acids, or at least 10 amino acids in length.

In a third aspect, the present disclosure provides an isolated polypeptide or an antigenic fragment thereof, wherein the polypeptide has an amino acid sequence selected from a group consisting of the following amino acid sequences or amino acid sequences obtained after a substitution, a deletion, an insertion and/or an addition of one or more amino acid residues has been made in the following amino acid sequences:
GWEIVKFISTCACEI (SEQ ID NO: 40);
WADNNCYLATALLTL (SEQ ID NO: 41);
KDYLASGGQPITNCV (SEQ ID NO: 42);
DKSAFVNLKQLPFFY (SEQ ID NO: 43);
FIEDLLFNKVTLADA (SEQ ID NO: 44);
LLLLDRLNQLESKMS (SEQ ID NO: 45);
LKKLKKSLNVAKSEF (SEQ ID NO: 46);
RLKLFAAETLKATEE (SEQ ID NO: 47); or
ASDTYACWHHSIGFD (SEQ ID NO: 48).

In some embodiments in the third aspect of the present disclosure, the antigenic fragment is at least 5 amino acids, at least 6 amino acids, at least 7 amino acids, at least 8 amino acids, at least 9 amino acids, or at least 10 amino acids in length.

In a fourth aspect, the present disclosure provides an isolated antibody, binding to the polypeptide or the antigenic fragment thereof according to the first aspect, the second aspect or the third aspect of the present disclosure.

In some embodiments, the antibody is selected from an IgG antibody, an IgM antibody and an IgA antibody. In some embodiments, the antibody is selected from an IgG1 antibody, an IgG2 antibody, an IgG3 antibody, and an IgG4 antibody. In some embodiments, the antibody is a monoclonal antibody. In some embodiments, the antibody is a human antibody.

In a fifth aspect, the present disclosure provides a composition, including one or more polypeptides or antigenic fragments thereof according to the first aspect, the second aspect or the third aspect of the present disclosure.

In some embodiments in the fifth aspect of the present disclosure, the composition includes a polypeptide or an antigenic fragment thereof having an amino acid sequence shown as SEQ ID NO: 10 and one or more of polypeptides or antigenic fragments thereof having amino acid sequences selected from amino acid sequences shown as SEQ ID NOs: 1-9 and 11-48. In some embodiments, the composition includes the polypeptide or the antigenic fragment thereof having the amino acid sequence shown as SEQ ID NO: 10 and 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, or 47 of the polypeptides or the antigenic fragments thereof having the amino acid sequences selected from amino acid sequences shown as SEQ ID NOs: 1-9 and 11-48.

In some embodiments, the composition includes the polypeptide or the antigenic fragment thereof having the amino acid sequence shown as SEQ ID NO: 10 and one or more of the polypeptides or the antigenic fragments thereof having the amino acid sequences selected from amino acid sequences shown as SEQ ID NOs: 1-9 and 11-39. In some embodiments, the composition includes the polypeptide or the antigenic fragment thereof having the amino acid sequence shown as SEQ ID NO: 10 and 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, or 38 of the polypeptides or the antigenic fragments thereof having the amino acid sequences selected from amino acid sequences shown as SEQ ID NOs: 1-9 and 11-39.

In some embodiments, the composition includes the polypeptide or the antigenic fragment thereof having the amino acid sequence shown as SEQ ID NO: 10 and one or more of the polypeptides or the antigenic fragments thereof having the amino acid sequences selected from amino acid sequences shown as SEQ ID NOs: 1-9 and 11-35. In some embodiments, the composition includes the polypeptide or the antigenic fragment thereof having the amino acid sequence shown as SEQ ID NO: 10 and 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, or 34 of the polypeptides or the antigenic fragments thereof having the amino acid sequences selected from amino acid sequences shown as SEQ ID NOs: 1-9 and 11-35.

In some embodiments, the composition includes the polypeptides or the antigenic fragments thereof having the amino acid sequences shown as SEQ ID NOs: 10, 7, 35, 29, 23, 30, 2, 21, and 12.

In some embodiments, the composition includes the polypeptides or the antigenic fragments thereof having the amino acid sequences shown as SEQ ID NOs: 10, 12, 35, 14, 30, 2, 3, 13, and 19.

In some embodiments, the composition includes the polypeptides or the antigenic fragments thereof having the amino acid sequences shown as SEQ ID NOs: 10, 35, 12, and 19.

In some embodiments, the composition includes the polypeptides or the antigenic fragments thereof having the amino acid sequences shown as SEQ ID NOs: 10 and 33.

In some embodiments, the composition includes the polypeptides or the antigenic fragments thereof having the amino acid sequences shown as SEQ ID NOs: 10, 32, and 15.

In some embodiments, the composition includes the polypeptides or the antigenic fragments thereof having the amino acid sequences shown as SEQ ID NOs: 10, 21, 30, 15, 12, 35, 23, 19, and 20.

In some embodiments, the antigenic fragment is at least 5 amino acids, at least 6 amino acids, at least 7 amino acids, at least 8 amino acids, at least 9 amino acids, or at least 10 amino acids in length.

In some embodiments, the composition is a vaccine composition, and optionally, further includes one or more adjuvants.

In a sixth aspect, the present disclosure provides a composition, including one or more antibodies according to the fourth aspect of the present disclosure, and an optional carrier and/or excipient.

In a seventh aspect, the present disclosure provides a nucleotide sequence, encoding the polypeptide or the antigenic fragment thereof according to the first aspect, the second aspect or the third aspect of the present disclosure, or the antibody according to the fourth aspect of the present disclosure. The present disclosure further provides a vector including the nucleotide sequence.

In an eighth aspect, the present disclosure provides an array, including a solid substrate and a polypeptide immobilized on the solid substrate. The polypeptide includes at least one polypeptide or antigenic fragment thereof according to the first aspect or the second aspect of the present disclosure.

In some embodiments, the polypeptide in the array includes at least 2, at least 3, at least 5, at least 10, at least 15, at least 20, at least 25, at least 30, or at least 35 polypeptides or antigenic fragments thereof according to the first aspect or the second aspect of the present disclosure.

In some embodiments, the polypeptide in the array includes a polypeptide or an antigenic fragment thereof having an amino acid sequence shown as SEQ ID NO: 10 and one or more of the polypeptides or the antigenic fragments thereof having the amino acid sequences selected from amino acid sequences shown as SEQ ID NOs: 1-9 and 11-39. In some embodiments, the polypeptide in the array includes the polypeptide or the antigenic fragment thereof having the amino acid sequence shown as SEQ ID NO: 10 and 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, or 38 of the polypeptides or the antigenic fragments thereof having the amino acid sequences selected from amino acid sequences shown as SEQ ID NOs: 1-9 and 11-39.

In some embodiments, the polypeptide in the array includes the polypeptide or the antigenic fragment thereof having the amino acid sequence shown as SEQ ID NO: 10 and one or more of the polypeptides or the antigenic fragments thereof having the amino acid sequences selected from amino acid sequences shown as SEQ ID NOs: 1-9 and 11-35. In some embodiments, the polypeptide in the array includes the polypeptide or the antigenic fragment thereof having the amino acid sequence shown as SEQ ID NO: 10 and 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, or 34 of the polypeptides or the antigenic fragments thereof having the amino acid sequences selected from amino acid sequences shown as SEQ ID NOs: 1-9 and 11-35.

In some embodiments, the polypeptide in the array includes the polypeptides or the antigenic fragments thereof having the amino acid sequences shown as SEQ ID NOs: 10, 7, 35, 29, 23, 30, 2, 21, and 12.

In some embodiments, the polypeptide in the array includes the polypeptides or the antigenic fragments thereof having the amino acid sequences shown as SEQ ID NOs: 10, 12, 35, 14, 30, 2, 3, 13, and 19.

In some embodiments, the polypeptide in the array includes the polypeptides or the antigenic fragments thereof having the amino acid sequences shown as SEQ ID NOs: 10, 35, 12, and 19.

In some embodiments, the polypeptide in the array includes the polypeptides or the antigenic fragments thereof having the amino acid sequences shown as SEQ ID NOs: 10 and 33.

In some embodiments, the polypeptide in the array includes the polypeptides or the antigenic fragments thereof having the amino acid sequences shown as SEQ ID NOs: 10, 32, and 15.

In some embodiments, the polypeptide in the array includes the polypeptides or the antigenic fragments thereof having the amino acid sequences shown as SEQ ID NOs: 10, 21, 30, 15, 12, 35, 23, 19, and 20.

In some embodiments, the antigenic fragment is at least 5 amino acids, at least 6 amino acids, at least 7 amino acids, at least 8 amino acids, at least 9 amino acids, or at least 10 amino acids in length.

In some embodiments, the polypeptide in the array includes a plurality of polypeptides or antigenic fragments thereof according to the first aspect or the second aspect of the present disclosure, where the plurality of polypeptides or antigenic fragments thereof include at least one polypeptide or antigenic fragment thereof derived from a SARS-CoV-2 ORF1ab polyprotein, and at least one polypeptide or antigenic fragment thereof derived from a SARS-CoV-2 S protein.

In some embodiments, the plurality of polypeptides further includes at least one polypeptide or antigenic fragment thereof derived from a SARS-CoV-2 N protein.

In a ninth aspect, the present disclosure provides an array, including a solid substrate and a polypeptide immobilized on the solid substrate. The polypeptide includes at least one polypeptide or antigenic fragment thereof according to the third aspect of the present disclosure.

In some embodiments, the polypeptide in the array includes at least 2, at least 3, at least 4, at least 5, at least 6, at least 7, at least 8, or at least 9 polypeptides or antigenic fragments thereof according to the third aspect of the present disclosure.

In some embodiments, the polypeptide in the array includes a plurality of polypeptides or antigenic fragments thereof according to the third aspect of the present disclosure, where the plurality of polypeptides or antigenic fragments thereof include at least one polypeptide or antigenic fragment thereof derived from the SARS-CoV-2 ORF1ab polyprotein, at least one polypeptide or antigenic fragment thereof derived from the SARS-CoV-2 S protein, and at least one polypeptide or antigenic fragment thereof derived from the SARS-CoV-2 N protein.

In some embodiments, the polypeptide is immobilized on the solid substrate after being coupled to BSA.

In some embodiments, the array is a polypeptide chip.

In a tenth aspect, the present disclosure provides a method for detecting the presence of an antibody against SARS-CoV-2 in a subject, including:
(a) contacting a sample from the subject with the polypeptide or the antigenic fragment thereof according to the first aspect or the second aspect of the present disclosure, or the array according to the eighth aspect or the ninth aspect of the present disclosure; and
(b) detecting the presence of the antibody in the sample specifically bound to the polypeptide or the polypeptide on the array.

In some embodiments, the method is used for diagnosing whether the subject is infected with SARS-CoV-2.

In an eleventh aspect, the present disclosure provides a method for the prognosis of the subject infected with SARS-CoV-2, including:
(a) contacting a sample from the subject with the polypeptide or the antigenic fragment thereof according to the third aspect of the present disclosure, or the array according to the ninth aspect of the present disclosure; and
(b) detecting the presence of an antibody in the sample specifically bound to the polypeptide or the polypeptide on the array.

In some embodiments, the antibody is an IgG antibody and/or an IgM antibody.

In some embodiments, the sample is a blood sample, for example, a serum or plasma.

In a twelfth aspect, the present disclosure provides a method for inducing an immune response against SARS-CoV-2 in a subject, including: administrating the polypeptide or the antigenic fragment thereof according to the first aspect, the second aspect or the third aspect of the present disclosure, or the composition according to the fifth aspect of the present disclosure to the subject.

In a thirteenth aspect, the present disclosure provides a method for preventing and/or treating a disease caused by SARS-CoV-2 infection in a subject, including: administrating the polypeptide or the antigenic fragment thereof according to the first aspect, the second aspect or the third aspect of the present disclosure, or the composition according to the fifth aspect of the present disclosure to the subject.

In some embodiments, the method includes: administrating a combination of at least 2, at least 3, at least 5, at least 10, at least 15, at least 20, at least 25, or at least 30 polypeptides or antigenic fragments thereof according to the first aspect, the second aspect or the third aspect of the present disclosure to the subject.

In some embodiments, the method includes: administrating a combination containing a polypeptide or an antigenic fragment thereof having an amino acid sequence shown as SEQ ID NO: 10 and one or more of polypeptides or antigenic fragments thereof having amino acid sequences selected from amino acid sequences shown as SEQ ID NOs: 1-9 and 11-48 to the subject. In some embodiments, the method includes: administrating a combination containing the polypeptide or the antigenic fragment thereof having the amino acid sequence shown as SEQ ID NO: 10 and 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, or 47 of the polypeptides or the antigenic fragments thereof having the amino acid sequences selected from amino acid sequences shown as SEQ ID NOs: 1-9 and 11-48 to the subject.

In some embodiments, the method includes: administrating a combination containing the polypeptide or the antigenic fragment thereof having the amino acid sequence shown as SEQ ID NO: 10 and one or more of the polypeptides or the antigenic fragments thereof having the amino acid sequences selected from amino acid sequences shown as SEQ ID NOs: 1-9 and 11-39 to the subject. In some embodiments, the method includes: administrating a combination containing the polypeptide or the antigenic fragment thereof having the amino acid sequence shown as SEQ ID NO: 10 and 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, or 38 of the polypeptides or the antigenic fragments thereof having the amino acid sequences selected from amino acid sequences shown as SEQ ID NOs: 1-9 and 11-39 to the subject.

In some embodiments, the method includes: administrating a combination containing the polypeptide or the antigenic fragment thereof having the amino acid sequence shown as SEQ ID NO: 10 and one or more of the polypeptides or the antigenic fragments thereof having the amino acid sequences selected from amino acid sequences shown as SEQ ID NOs: 1-9 and 11-35 to the subject. In some embodiments, the method includes: administrating a combination containing the polypeptide or the antigenic fragment thereof having the amino acid sequence shown as SEQ ID NO: 10 and 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, or 34 of the polypeptides or the antigenic fragments thereof having the amino acid sequences selected from amino acid sequences shown as SEQ ID NOs: 1-9 and 11-35 to the subject.

In some embodiments, the method includes: administrating a combination containing the polypeptides or the antigenic fragments thereof having the amino acid sequences shown as SEQ ID NOs: 10, 7, 35, 29, 23, 30, 2, 21, and 12 to the subject.

In some embodiments, the method includes: administrating a combination containing the polypeptides or the antigenic fragments thereof having the amino acid sequences shown as SEQ ID NOs: 10, 12, 35, 14, 30, 2, 3, 13, and 19 to the subject.

In some embodiments, the method includes: administrating a combination containing the polypeptides or the antigenic fragments thereof having the amino acid sequences shown as SEQ ID NOs: 10, 35, 12, and 19 to the subject.

In some embodiments, the method includes: administrating a combination containing the polypeptides or the antigenic fragments thereof having the amino acid sequences shown as SEQ ID NOs: 10 and 33 to the subject.

In some embodiments, the method includes: administrating a combination containing the polypeptides or the antigenic fragments thereof having the amino acid sequences shown as SEQ ID NOs: 10, 32, and 15 to the subject.

In some embodiments, the method includes: administrating a combination containing the polypeptides or the antigenic fragments thereof having the amino acid sequences shown as SEQ ID NOs: 10, 21, 30, 15, 12, 35, 23, 19, and 20 to the subject.

In some embodiments, the antigenic fragment is at least 5 amino acids, at least 6 amino acids, at least 7 amino acids, at least 8 amino acids, at least 9 amino acids, or at least 10 amino acids in length.

In some embodiments, the method includes: administrating a plurality of polypeptides or antigenic fragments thereof according to the first aspect, the second aspect, or the third aspect of the present disclosure to the subject, where the plurality of polypeptides or antigenic fragments thereof include at least one polypeptide or antigenic fragment thereof derived from a SARS-CoV-2 ORF1ab polyprotein, at least one polypeptide or antigenic fragment thereof derived from a SARS-CoV-2 S protein, and at least one polypeptide or antigenic fragment thereof derived from a SARS-CoV-2 N protein.

In a fourteenth aspect, the present disclosure provides a method for treating a disease caused by SARS-CoV-2 infection in a subject, including: administrating the antibody according to the fourth aspect of the present disclosure to the subject.

### BRIEF DESCRIPTION OF DRAWINGS

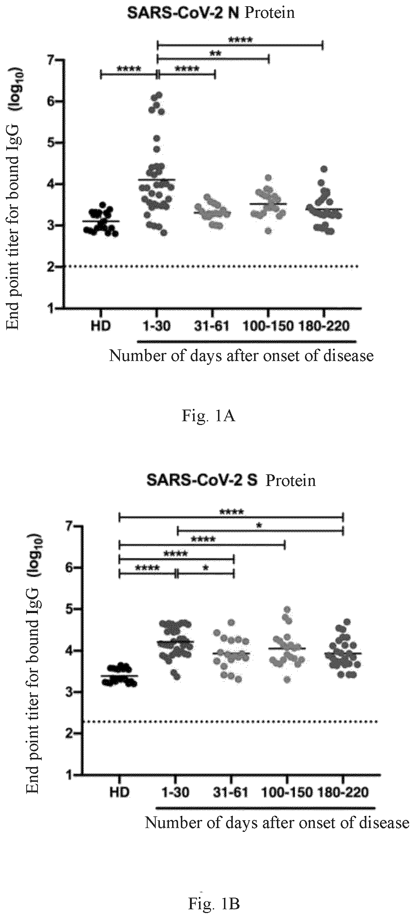

Fig. 1A and Fig. 1B show titers of an antigen-specific IgG antibody against a SARS-CoV-2 N protein (A) or S protein (B) in serum samples at different time points after the onset of symptoms of SARS-CoV-2 infection (on Days 1-30, n = 36; on Days 31-61, n = 18; on Days 100-150, n = 21; and on Days 180-220, n = 26), where a control group is serum samples of 20 healthy donors (HDs).

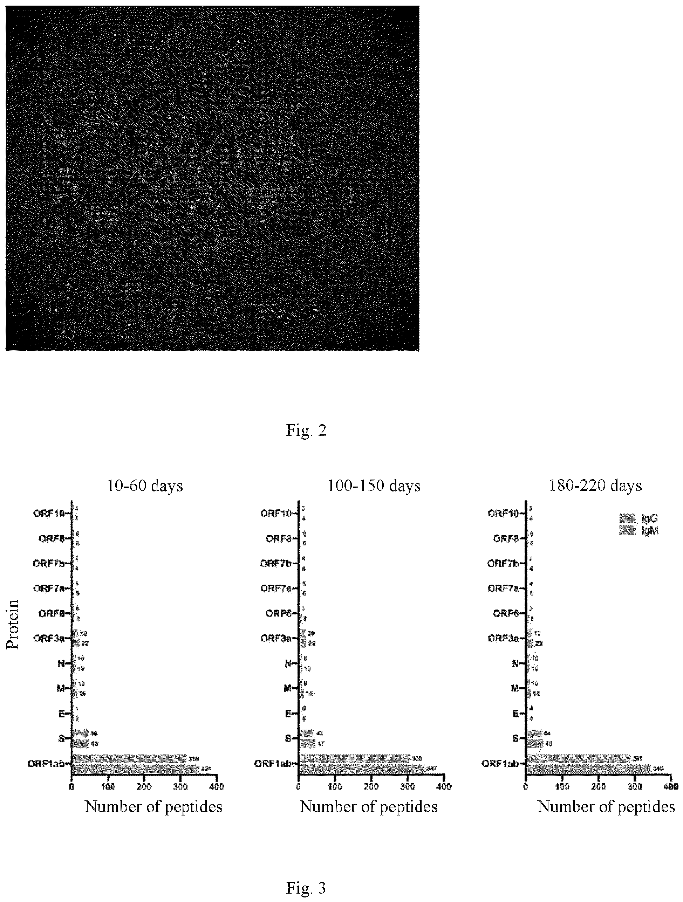

Fig. 2 is a schematic diagram of detecting serum samples using polypeptide chips, where peptides in a polypeptide pool are linked to BSA and then printed on the chips. After addition of the serum samples for reactions, the chips are scanned with LuxScan10K-A (Beijing CapitalBio, China). Parameters are set to 95% laser power/PMT550 and 95% laser power/PMT480 respectively. Fluorescence intensity data is obtained by GenePixPro6.0 software (Molecular Devices, CA, USA), where green fluorescence indicates an IgG intensity, red fluorescence indicates an IgM intensity, and yellow is superposition of green and red.
Fig. 3 shows counts and distribution of a peptide to which a positive response is detectable in at least one sample collected on Days 10-60 (n = 18), Days 100-150 (n = 18), and Days 180-220 (n = 15) after the onset of the disease, where numbers represent total numbers of IgG and IgM epitopes identified from each protein; and abbreviations: ORF, open reading frame; S, spike protein; E, envelope protein; N, nucleocapsid protein; and M, membrane protein.

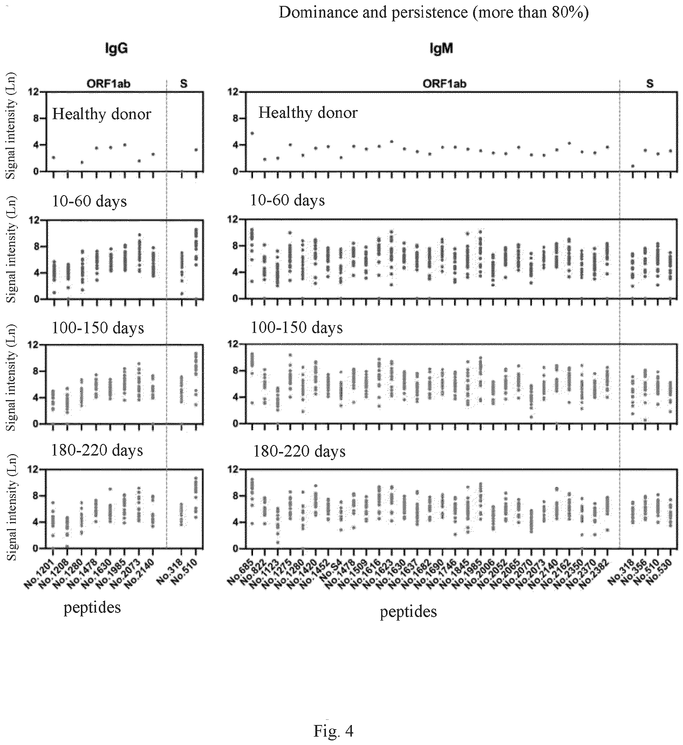

Fig. 4 shows signal intensities of dominant and persistent IgG and IgM epitopes, where each point represents a mixed serum from a healthy donor or a serum sample from a different patient collected at a specified time point after the onset of symptoms.

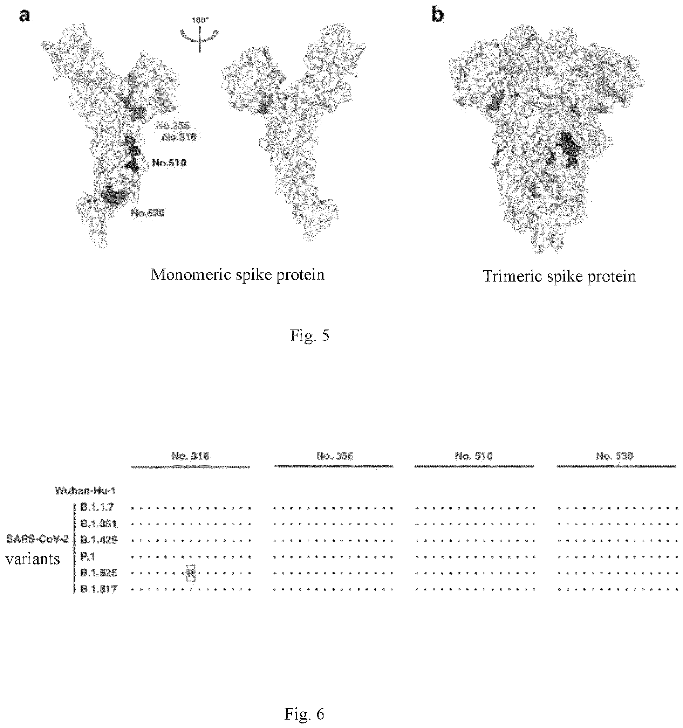

Fig. 5 is a schematic diagram of positions of dominant and persistent epitopes on 3D structures of monomeric (a) and trimeric (b) S proteins (PBD ID: 6VXX).
Fig. 6 shows conservative analysis on epitopes of early SARS-CoV-2 (a Wuhan-Hu-1 strain) and six emerging variant strains, where black points represent same amino acid residues as the Wuhan-Hu-1 strain.

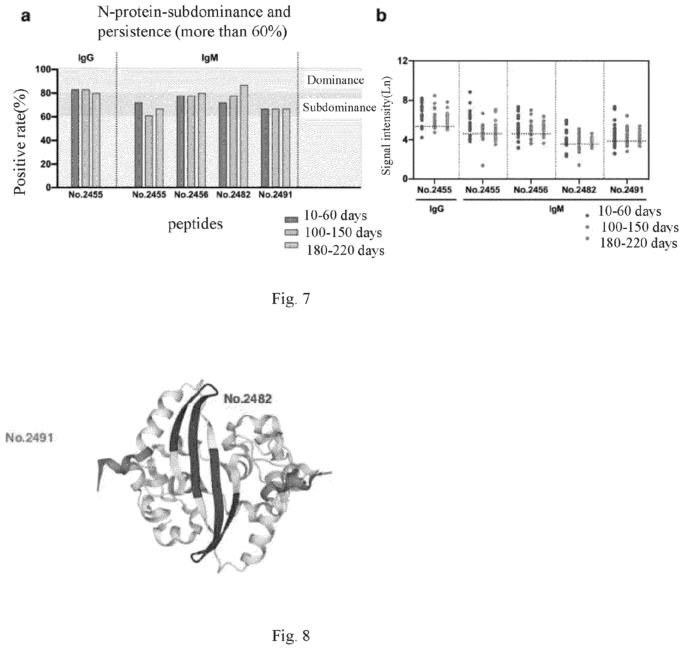

Fig. 7: a shows IgG and IgM recognition frequencies of subdominant epitopes in serum samples, collected at multiple time points after the onset of a disease, of patients; and b shows signal intensity kinetics of the identified subdominant epitopes over time, where each point represents a serum sample from a different patient obtained from an individual with COVID-19 at a specified time point after the onset of symptoms; and each dotted line represents a cut-off value of a positive response of each peptide.
Fig. 8 shows detailed structural analysis on epitopes of a C-terminal dimerization domain of a N protein (PDB ID: 6YUN).

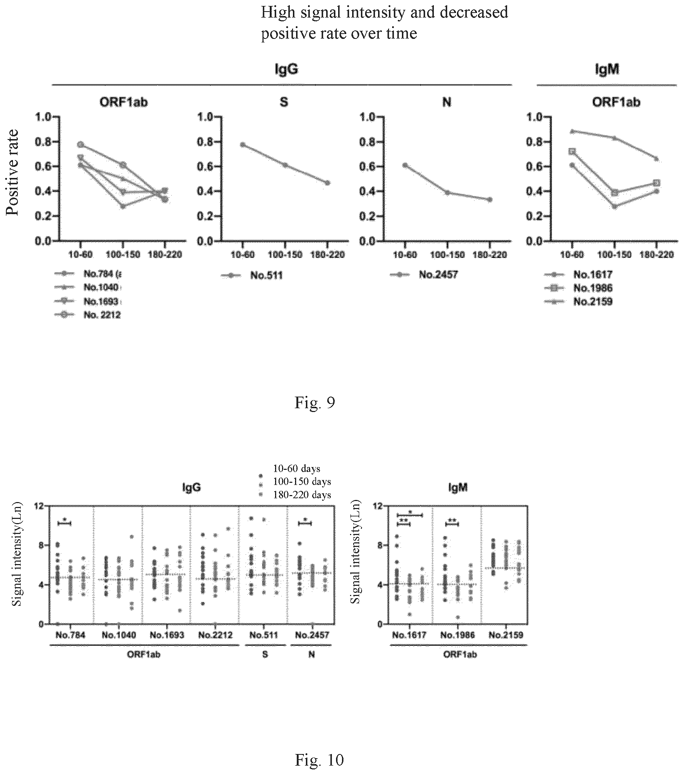

Fig. 9 shows that recognition frequencies (positive rates) of high binding signal intensity peptides are lowered over time.
Fig. 10 shows signal intensities of epitopes at three sampling time points after the onset of symptoms, where each point represents a serum, collected at a specified time point after the onset of the symptoms, of an individual patient, and each dotted line represents a cut-off value of a positive response of each peptide; and "*" represents p < 0.05, and "**" represents p < 0.01.

### DETAILED DESCRIPTION

The present disclosure relates to an isolated polypeptide or an antigenic fragment thereof, an antibody binding to the isolated polypeptide or the antigenic fragment thereof, a composition including the isolated polypeptide or the antigenic fragment thereof, and an array including the isolated polypeptide or the antigenic fragment thereof. The present disclosure further provides a method for detecting the presence of an antibody against SARS-CoV-2 in a subject, a method for the prognosis of the subject infected with SARS-CoV-2, a method for inducing an immune response against SARS-CoV-2 in a subject, and a method for preventing and/or treating a disease caused by SARS-CoV-2 infection in a subject.

Any aspect or embodiment described herein can be combined with any other aspect or embodiment disclosed herein. Although the present disclosure has been described in combination with the detailed description of the present disclosure, the previous description aims to explain rather than limit the scope of the present disclosure, and the scope of the present disclosure is limited by the scope of the appended claims. Other aspects, advantages and modifications fall within the scope of the appended claims. Patent and scientific literature referred to herein establishes the knowledge that is available to those skilled in the art. All US patents and published or unpublished US patent applications cited herein are incorporated herein by reference. All published foreign patents and patent applications cited herein are incorporated herein by reference. All other published references, documents, manuscripts and scientific literatures cited herein are incorporated herein by reference. Other features and advantages of the present disclosure will be apparent through the following detailed descriptions, including the embodiments and the claims.

### Definition

In order to more easily understand disclosure of the present invention, certain terms are first defined below. Additional definitions of the following terms and other terms are described throughout the specification.

Unless otherwise specified, all technical and scientific terms used herein shall have the same meaning as those commonly understood by that of ordinary skill in the art. For example, the terms used herein are defined as in Immunobiology by Janeway CA Jr, Travers P, Walport M et al., Fifth Edition, New York: GarlandScience (2001), and A multilingual glossary of bio terms: (IUPAC Recommendations), edited by Leuenberger. H.G.W, Nagel, B. nd Kölbl, H. (1995), Helvetica Chimica Acta, CH-4010 Basel, Switzerland.

It should be noted that the singular forms "a", "an" and "the/said" of the terms used herein and in the appended claims include plural referents, unless the context clearly specifies otherwise. Therefore, the terms "a", "an", "one or more" and "at least one" may be used interchangeably. Similarly, the terms "contain", "include" and "have" may be used interchangeably.

When the term "contain" or "comprise" is used herein and in the appended claims, it does not exclude other elements. For the purposes of the present invention, the term "consist of" is considered to be the preferred embodiment of the term "contain" or "comprise".

The terms "polypeptide", "peptide", and "protein" may be used interchangeably. They refer to compounds composed of amino acid residues covalently linked by peptide bonds without limiting a minimum length of a product. Therefore, the above terms include the peptide, an oligopeptide, the polypeptide, a dimer (heterologous and homologous), a polymer (heterologous and homologous), etc. The "isolated" polypeptide refers to a polypeptide extracted from its natural environment, or a polypeptide artificially synthesized by using a standard molecular biology technology and an expression system. For example, the polypeptide of the present disclosure may be isolated from a wild-type or mutant SARS-CoV-2 viral protein, or chemically synthesized by using a commercially automatic method (for example, completely by solid phase synthesis, partially by a solid phase synthesis method, a fragment combination method, or solution synthesis).

The "protein" and the "polypeptide" cover full-length proteins and their fragments. The term further includes post-expression modifications (such as glycosylation, acetylation, and phosphorylation) of the polypeptide. In addition, for the purposes of the present disclosure, the polypeptide further refers to a variant obtained by modifying an amino acid sequence of a natural protein or polypeptide.

Typically, modifications on the amino acid sequence include substitution, deletion, insertion, and addition. As used herein, "substitution" means that at least one residue has been removed, and a different residue is inserted into that position. Amino acid substitution usually involves a single residue, but may occur at many different positions simultaneously. "Deletion" is characterized by removal of one or more amino acid residues from the sequence. Usually, no more than about 2-6 residues are deleted from any site within a protein molecule. "Insertion" refers to insertion of single or multiple amino acid residues within the sequence, while "addition" refers to amino-and/or carboxyl-terminal fusions. Compared with addition, insertion usually refers to small addition, for example, of about 1-4 residues.

The term "variant" of an amino acid sequence includes any substitution, change, modification, substitution, deletion, or addition from the sequence or of one (or more) amino acid in the sequence. A single variant may contain substitution, deletion, insertion, addition, or any combination thereof, as long as the polypeptide variant has antigenicity.

"Immunogenicity", also known as "antigenicity" and "immunoreactivity", refers to an ability of the protein or the polypeptide to produce an antibody or induce an antibody response in a subject. An "antigenic fragment" refers to a polypeptide fragment that can induce an immune reaction. The antigenic fragment may be, for example, at least 5 amino acids, at least 6 amino acids, at least 7 amino acids, at least 8 amino acids, at least 9 amino acids, or at least 10 amino acids in length, for example, 5, 6, 7, 8, 9, 10, 11, 12, 13, 14, or 15 amino acids in length. The antigenic fragments may be produced by a method known to those skilled in the art.

Herein, an "epitope" or an "antigenic epitope" may be used interchangeably, being the term in the art, and refers to a local region of an antigen to which the antibody may specifically bind. The epitope may be, for example, continuous amino acids (linear or continuous epitopes) of the polypeptide, or two or more non-continuous regions (conformational, non-linear, discontinuous or non-continuous epitopes) from one or more polypeptides.

In this context, the term "bind" or "specifically bind" usually refers to a noncovalent association between two or more entities, which is defined as the antibody binding to the antigen by its affinity (KD) of less than approximately 10⁻⁵ M, for example, less than approximately 10⁻⁶ M, 10⁻⁷ M, 10⁻⁸ M, 10⁻⁹ M, 10⁻¹⁰ M, or less, with a corresponding epitope. "KD" used herein refers to a dissociation equilibrium constant of a specific antibody-antigen interaction, which is used for describing the binding affinity between the antibody and the antigen. The smaller the equilibrium dissociation constant is, the closer the antibody-antigen binding is, and the higher the affinity between the antibody and the antigen is. Measurement on a binding constant is known to those skilled in the art.

As used herein, the "antibody" is an immunoglobulin molecule that can specifically bind to a target through at least one antigen recognition site located in a variable domain of the immunoglobulin molecule. The antibody mentioned herein should be understood in its broadest sense. The antibody includes any class of antibodies, such as IgD, IgE, IgG, IgA or IgM (or its subclasses), and does not have to be of any particular class. Any antibody involved in the present disclosure may be monoclonal or polyclonal. The term "monoclonal antibody" refers to a homogeneous antibody population, while the term "polyclonal antibody" refers to a heterogeneous antibody population. These two terms should not be interpreted as limiting to a source or preparation method of the antibody.

The term "neutralization" refers to an antigen-binding molecule, scFv, an antibody, or a fragment thereof that binds to a ligand and prevents or weakens biological effects of the ligand. In some embodiments, the antigen-binding molecule, scFv, the antibody or the fragment thereof directly block a binding site on the ligand or indirectly change a binding ability of the ligand (for example, a structural or energy change of the ligand). In some embodiments, the antigen-binding molecule, scFv, the antibody or the fragment thereof prevents the protein binding thereto from performing biological functions.

The terms "nucleic acid", "nucleotide", and "nucleotide sequence" may be used interchangeably, and refer to an oligomer or a polymer in any length that is basically composed of nucleotides (such as deoxyribonucleotides and/or ribonucleotides). The nucleic acid may contain purine and/or a pyrimidine base and/or other natural (for example, xanthine, inosine, and hypoxanthine), chemically or biochemically modified (for example, methylated), unnatural or derived nucleotide bases. A backbone of the nucleic acid may contain sugars and phosphate groups normally present in RNA or DNA, and/or one or more modified or substituted sugars, and/or one or more modified or substituted phosphate groups. Modifications on the phosphate groups or the sugars may be introduced to improve stability, resistance to enzyme-catalyzed degradation, or some other useful properties. The "nucleic acid" may be, for example, double-stranded, partially double-stranded or single-stranded. When it is single-stranded, the nucleic acid may have a sense strand or an antisense strand. The "nucleic acid" may be circular or linear. As used herein, the term "nucleic acid" covers DNA and RNA, including a genome, pre-mRNA, mRNA, cDNA, a recombinant or synthetic nucleic acid containing a vector. For the purposes of the uses herein, it should be understood that a polynucleotide may be modified by any method available in the art.

Those skilled in the art will appreciate that many different polynucleotides and nucleic acids may encode a same polypeptide due to degeneracy of a genetic code. Moreover, it should be understood that those skilled in the art may use a conventional technology to perform substitutions of the nucleotides without affecting the polypeptide sequences encoded by the polynucleotides described herein, so as to reflect the use of a codon in any specific host organism with the polypeptide to be expressed therein.

As used herein, the term "infection" means invasion through proliferation and/or presence of viruses in cells or individuals. In some embodiments, infection refers to "active" infection, that is, infection of virus replication in the cells or the individuals. Such infection is characterized by spreading the viruses from the cells, tissues, and/or organs first infected with them to other cells, tissues and/or organs. Infection may also refer to latent infection, that is, infection without virus replication. In some embodiments, infection refers to a pathological state caused by the viruses present in the cells or the individuals, or a pathological state caused by invasion of the cells or the individuals by the viruses.

### Antigenic polypeptide and variant thereof

The protein and the polypeptide herein may refer to a variant of the natural protein or polypeptide, having at least 50%, at least 55%, at least 60%, at least 65%, at least 70%, at least 75%, at least 80%, at least 85%, at least 90%, at least 95%, at least 98%, or at least 99% sequence identity to the natural protein or polypeptide, provided that the variant maintains initial functions or activity of the natural protein or polypeptide.

The present disclosure provides an isolated polypeptide or an antigenic fragment thereof. The polypeptide has an amino acid sequence selected from any one of SEQ ID NOs: 1-39. In some embodiments, the present disclosure provides an isolated polypeptide or an antigenic fragment thereof. The polypeptide includes an amino acid sequence having at least 50%, for example, at least 60%, at least 70%, at least 80%, at least 90%, or at least 100%, for example, 50%, 55%, 60%, 65%, 70%, 75%, 80%, 85%, 90%, 91%, 92%, 93%, 94%, 95%, 96%, 97%, 98%, 99%, or 100%, identity to an amino acid sequence selected from any one of SEQ ID NOs: 1-39. In some embodiments, the antigenic fragment of the polypeptide is at least 5 amino acids, at least 6 amino acids, at least 7 amino acids, at least 8 amino acids, at least 9 amino acids, or at least 10 amino acids in length, for example, 5, 6, 7, 8, 9, 10, 11, 12, 13, 14, or 15 amino acids in length.

The present disclosure further provides an isolated polypeptide or an antigenic fragment thereof. The polypeptide has an amino acid sequence selected from any one of SEQ ID NOs: 1-35. In some embodiments, the present disclosure provides an isolated polypeptide or an antigenic fragment thereof. The polypeptide includes an amino acid sequence having at least 50%, for example, at least 60%, at least 70%, at least 80%, at least 90%, or at least 100%, for example, 50%, 55%, 60%, 65%, 70%, 75%, 80%, 85%, 90%, 91%, 92%, 93%, 94%, 95%, 96%, 97%, 98%, 99%, or 100%, identity to an amino acid sequence selected from any one of SEQ ID NOs: 1-35. In some embodiments, the antigenic fragment of the polypeptide is at least 5 amino acids, at least 6 amino acids, at least 7 amino acids, at least 8 amino acids, at least 9 amino acids, or at least 10 amino acids in length, for example, 5, 6, 7, 8, 9, 10, 11, 12, 13, 14, or 15 amino acids in length.

The present disclosure further provides an isolated polypeptide or an antigenic fragment thereof. The polypeptide has an amino acid sequence selected from any one of SEQ ID NOs: 40-48. In some embodiments, the present disclosure provides an isolated polypeptide or an antigenic fragment thereof. The polypeptide includes an amino acid sequence having at least 50%, for example, at least 60%, at least 70%, at least 80%, at least 90%, or at least 100%, for example, 50%, 55%, 60%, 65%, 70%, 75%, 80%, 85%, 90%, 91%, 92%, 93%, 94%, 95%, 96%, 97%, 98%, 99%, or 100%, identity to an amino acid sequence selected from any one of SEQ ID NOs: 40-48. In some embodiments, the antigenic fragment of the polypeptide is at least 5 amino acids, at least 6 amino acids, at least 7 amino acids, at least 8 amino acids, at least 9 amino acids, or at least 10 amino acids in length, for example, 5, 6, 7, 8, 9, 10, 11, 12, 13, 14, or 15 amino acids in length.

As used herein, the "sequence identity" refers to a degree of identity between any given query sequence and a subject sequence. Those skilled in the art will readily understand how to determine the identity between two polypeptides (for example, an unmodified polypeptide and a polypeptide variant). For example, the identity may be calculated after two sequences are aligned with their identity reaching the highest level, for example, by introducing a gap. Another method for calculating the identity may be implemented through a public algorithm. Non-limiting examples of such mathematical algorithms include an algorithm of Myers and Miller (1988) (CABIOS 4: 11-17), a local homology algorithm of Smith et al. (1981) (Adv. Appl. Math. 2: 482), a homology alignment algorithm of Needleman and Wunsch (1970) (J. Mol. Biol. 48: 443-453), a method for searching homology, Pearson and Lipman (1988) (Proc. Natl. Acad. Sci. 85: 2444-2448), a modified form of an algorithm of Karlin and Altschul (1990) (Proc. Natl. Acad. Sci. USA 87: 2264), and an algorithm described in Karlin and Altschul (1993) in Proc. Natl. Acad. Sci. USA 90: 5873-5877. Sequence comparison (i.e., alignment) for determining the sequence identity may be implemented by using programs based on such mathematical algorithms. The programs may be properly executed by a computer. Examples of such programs include, but not limited to, CLUSTAL and ALIGN programs (Version 2.0) of the PC/Gene program, and GAP, BESTFIT, BLAST, FASTA, and TFASTA in the Wisconsin genetics software package. For example, alignment of these programs may be implemented by using initial parameters.

In some embodiments, the isolated polypeptide or the antigenic fragment thereof of the present disclosure include a variant, which may contain one or more amino acid modifications. A single variant may contain substitution, deletion, insertion, addition, or any combination thereof, as long as the polypeptide variant has antigenicity. A method and a technology for preparing the variant are well known by those skilled in the art.

The amino acid substitution used herein may be a conservative or non-conservative substitution. In this regard, it is understood in the art that the amino acids may be grouped based on their physical properties. Examples of such groups include, but not limited to, charged amino acids, uncharged amino acids, polar uncharged amino acids, and hydrophobic amino acids. The conservative amino acid substitution may involve substituting the natural amino acid residues with the non-natural residues, so that there is little or no effect on size, polarity, charge, hydrophobicity or hydrophilicity of the amino acid residues at this position, specifically, it does not lead to weakening of the antigenicity.

Herein, substitution with conservative amino acids refers to exchangeability with residues having similar side chains. Families of amino acid residues having similar side chains have been defined in the art. These families include amino acids having the following side chains: basic side chains (for example, lysine, arginine, and histidine), acidic side chains (for example, aspartic acid and glutamic acid), uncharged polar side chains (for example, glycine, asparagine, glutamine, serine, threonine, tyrosine, cysteine, and tryptophan), non-polar side chains (for example, alanine, valine, leucine, isoleucine, proline, phenylalanine, and methionine), beta branched side chains (for example, the threonine, the valine, and the isoleucine), and aromatic side chains (for example, the tyrosine, the phenylalanine, the tryptophan, and the histidine).

In some embodiments, the substitution is not conservative. For example, amino acids capable of changing some properties or aspects of the polypeptide may be used for substitution. In some embodiments, the non-conservative amino acid substitution may be performed, for example, with changing a structure of the polypeptide, and changing binding properties of the polypeptide (for example, strengthening or weakening binding affinity of the polypeptide, and/or strengthening or weakening binding specificity of the polypeptide).

### Antibody

The present disclosure further provides an isolated antibody, binding to the polypeptide or the antigenic fragment thereof of the present disclosure. As used herein, the term "antibody" is understood in its broadest sense. It not only covers intact (i.e., full-length) polyclonal or monoclonal antibodies, but also antigen-binding fragments thereof (such as Fab, Fab', F (ab')₂, Fv), single chain (scFv), mutants thereof, fusion proteins containing antibody portions, humanized antibodies, chimeric antibodies, double antibodies, nanobodies, linear antibodies, single-chain antibodies, multi-specific antibodies (for example, bispecific antibodies), and any other modified configuration of the immunoglobulin molecule containing required specific antigen recognition sites, including glycosylation variants of the antibodies, amino acid sequence variants of the antibodies, and covalently modified antibodies. The antibody includes any class of antibodies, such as IgD, IgE, IgG, IgA or IgM (or its subclasses), and does not have to be of any particular class. Immunoglobulins may be divided into different classes according to the antibody amino acid sequences of their heavy chain constant domains. There are five main classes of immunoglobulins: IgA, IgD, IgE, IgG, and IgM, and several of these immunoglobulins may be further divided into subclasses (isotypes), for example, IgG1, IgG2, IgG3, and IgG4. The heavy chain constant domains corresponding to different classes of immunoglobulins are called α, δ, ε, γ, and µ respectively. Subunit structures and three-dimensional configurations of different classes of immunoglobulins are well known.

Any antibody described herein may be monoclonal or polyclonal. As used herein, the term "monoclonal antibody" refers to an antibody obtained from a population of substantially homogeneous antibodies, that is, various antibodies constituting the population are identical, except for possible naturally occurring mutations that may be present in minor amounts. The monoclonal antibodies are highly specific, being directed against a single antigen. In addition, in contrast to polyclonal antibody formulations usually including different antibodies directed against different determinants (epitopes), each antibody in a monoclonal formulation is directed against a same single determinant on the antigen. The monoclonal antibodies are not limited to antibodies produced through a hybridoma technology. The term "polyclonal antibody" refers to a heterogeneous antibody population, which is a mixture of antibodies containing various types of antibodies, and usually produced by different types of plasma cells. These two terms should not be interpreted as limiting to a source or preparation method of the antibody.

For example, in some embodiments, the monoclonal antibodies include the chimeric antibodies, such as humanized versions of murine monoclonal antibodies. Such humanized antibodies may be prepared by known technologies, and offer the advantage of weakening the immunogenicity when being administrated to humans. In one embodiment, humanized monoclonal antibodies contain variable domains (or only their antigen binding sites) of the murine antibodies and constant domains derived from the human antibodies. Alternatively, humanized antibody fragments may contain antigen binding sites of murine monoclonal antibodies and variable domain fragments derived from the human antibodies (lack of the antigen binding sites). The programs for producing chimeric monoclonal antibodies and further engineered monoclonal antibodies include the following described programs: Riechmann et al. (Nature 332:323, 1988), Liu et al. (PNAS 84:3439, 1987), Larrick et al. (Bio/Technology 7:934, 1989), and Winter and Harris (TIPS 14:139, May, 1993). The programs for transgenically producing the antibodies may be found in GB 2,272,440, and U.S. Patent Nos. 5,569,825 and 5,545,806.

The antibodies (such as chimeric and humanized monoclonal antibodies) produced by genetic engineering methods may be used. The antibodies contain both human and non-human portions, and may be made by using a standard recombinant DNA technology. Such chimeric and humanized monoclonal antibodies may be produced by genetic engineering using a standard DNA technology known in the art.

Human monoclonal antibodies with human constant and variable domains may be produced by immunizing transgenic animals containing human immunoglobulin genes. See Jakobovits et al., Ann NY Acad Sci 764: 525-535 (1995). The human monoclonal antibodies directed against a PLA2-GIB polypeptide may also be prepared by constructing a combinatorial immunoglobulin pool (such as a Fab phage display pool or a scFv phage display pool) using immunoglobulin light chain and heavy chain cDNAs prepared from mRNA derived from subject's lymphocytes. See, for example, McCafferty et al., PCT Publication WO 92/01047, Marks et al., (1991) J. Mol. Biol. 222: 581 597, and Griffths et al., (1993) EMBO J 12: 725 734. Moreover, a combinatorial pool of antibody variable domains may be generated by mutating known human antibodies. For example, a pool of mutational variable domains may be generated by mutating known variable domains, binding to PLA2-GIB, of the human antibodies, for example, using randomly changed mutagenic oligonucleotides, and then may be screened to make the variable domains bind to PLA2-GIB. A method of inducing random mutagenesis in a CDR region of immunoglobulin heavy chain and/or light chain, a method for hybridization of random heavy chain and light chain to form pairing, and a screening method may be found in for example, Barbas et al., PCT published WO 96/07754, and Barbas et al., (1992) Proc. Nat'l Acad. Sci. USA 89: 4457 4461.

Antibodies with a strong neutralizing capability may provide therapeutic and prophylactic agents against COVID-19. However, only focusing on the S protein and a RBD region of the S protein may put more evolutionary selection pressure on this region, so that mutations in this region are enriched, which weakens effectiveness of therapeutic antibodies and vaccines centered in the RBD region (Wang, L., et al., Importance of Neutralizing Monoclonal Antibodies Targeting Multiple Antigenic Sites on the Middle East Respiratory Syndrome Coronavirus Spike Glycoprotein To Avoid Neutralization Escape. J Virol, 2018. 92 (10)).

In fact, it is known that in addition to the RBD region, other regions or epitopes of the S protein may also elicit neutralizing antibodies. Other proteins, such as the N protein, the Orf9b protein, and the Nsp5 protein, may also elicit a significant antibody neutralization response. In addition, there are also significant differences in antibody responses induced by an E protein and the Orf7b protein between a healthy population and a virus infected population (Jiang, H.W., et al., SARS-CoV-2 proteome microarray for global profiling of COVID-19 specific IgG and IgM responses. Nat Commun, 2020. 11 (1): p. 3581).

### Nucleotide and vector

The present disclosure further provides a nucleotide sequence, encoding the polypeptide or the antigenic fragment thereof according to the present disclosure, or the antibody according to the present disclosure. In another aspect, the present disclosure further relates to a vector according to a nucleotide sequence of the present disclosure.

The "vector" used herein refers to a nucleic acid delivery vehicle to which a nucleotide can be inserted. When the vector permits expression of a protein encoded by the inserted nucleotide, it is called an expression vector. The vector can introduce a host cell by transformation, transduction, transfection or other methods, and then a genetic material element carried therein is expressed in the host cell. The vector is recognized by those skilled in the art, including, but not limited to: (1) a plasmid; (2) phagemid; (3) cosmid; (4) an artificial chromosome such as a yeast artificial chromosome, a bacterial artificial chromosome or a P1-derived artificial chromosome; (5) a phage such as a λ phage or an M13 phage; and (6) an animal virus such as a retrovirus, an adenovirus, adeno-associated virus, a herpes virus, a poxvirus, and a baculovirus. A vector may contain a plurality of elements controlling expression, including, but not limited to, a promoter sequence, a transcription initiation sequence, an enhancer sequence, a selection element, and a reporter gene; and in addition, the vector may further contain an origin of replication.

### SARS-CoV-2 virus and COVID-19

SARS-CoV-2 virus, i.e. severe acute respiratory syndrome coronavirus 2, is an enveloped, positive-strand single-stranded RNA virus, belonging to severe acute respiratory syndrome-related coronavirus species of Betacoronavirus of the family Coronaviridae. Gene sequences of the SARS-CoV-2 virus, the SARS virus, and the MERS virus belong to a same lineage, but are different in evolutionary branch; and the SARS-CoV-2 virus is the seventh known coronavirus capable of infecting the humans. Hosts of the virus include mammals and poultry. The virus can invade a human body through a human upper respiratory tract, and achieve an infection with ACE2 expressed on surfaces of various cells as a receptor. Main infected organs include lung, heart, kidney and other major organs. At present, a mainstream detection method for a SARS-CoV-2 case is RT-PCR. The "SARS-CoV-2 virus" and the "novel coronavirus" may be used interchangeably herein.

A genome sequence of the SARS-CoV-2 virus is 5'UTR-ORF1a /b-structural protein gene-3'UTR. It is predicted that the SARS-CoV-2 virus can encode 29 proteins, including 4 structural proteins, 9 accessory proteins, and 16 non-structural proteins (Nsp). The structural proteins include a membrane protein (M protein), a nucleocapsid protein (N protein), a spike protein (S protein), and an envelope protein (E protein). Among them, the non-structural protein sequence of the SARS-CoV-2 virus is relatively conservative. The ORF1ab gene encodes a multifunctional protein involved in viral RNA transcription and replication, including a protease responsible for cutting a polyprotein. The S protein belongs to the trimeric class I fusion protein, including two subunits S1 and S2 and a transmembrane anchor. The S1 subunit contains one signal peptide, an N-terminal domain (NTD), and a receptor binding domain (RBD). The S2 subunit contains a fusion peptide (FP), heptad repeats (HR) 1 and 2, a transmembrane domain, and an intracellular domain. The N protein has a highly conservative sequence which plays an important role in viral replication. The N protein binds to the viral RNA to form a complex, which is then encapsulated into a viral capsid under the combined action of the M protein and the E protein. The N protein contains N1 and N2 epitopes. The epitope N1 can stimulate the body to produce a high-affinity antibody which, however, generally has no neutralizing activity.

The SARS-CoV-2 virus will cause an infectious disease COVID-19 (coronavirus disease 2019, also known as "novel coronavirus pneumonia"). Its common clinical symptoms include a fever, a cough, and shortness of breath, and some symptoms such as a sore throat, muscle weakness, and phlegm accumulated could be seen. Pathological researches on COVID-19 have shown that the SARS-CoV-2 virus infection will cause a respiratory system damage (such as an acute lung lesion), a cytokine release syndrome (such as cytokine storms and an acute inflammatory response), a circulatory system damage (such as a cardiovascular damage such as arrhythmia, vascular embolism, and pulmonary vasoconstriction), a digestive system damage (such as pancreas, liver and other organs), a genitourinary system damage (such as an acute renal failure), a nervous system damage (such as loss of smell and taste), an eye damage (such as conjunctival infection), etc. In addition, some patients with COVID-19 will still experience a series of effects (long-term syndrome of the coronavirus disease 2019) after rehabilitation.

### Composition and vaccine composition

The present disclosure provides a composition, including one or more polypeptides or antigenic fragments thereof of the present disclosure. In some embodiments, the composition is a vaccine composition. The "vaccine composition" is a composition that induces or enhances an immune response against an antigenic substance contained in the composition when being administrated to a subject (for example, a mammal). This reaction may include induction of antibody production (for example, by stimulating B cells) or a T cell-based reaction (for example, a cytolytic reaction). These reactions may or may not be protective or neutralizing. Protective or neutralizing immune reactions are immune reactions that are harmful to infected organisms that are consistent with antigens (such as organisms from which the antigens are derived), but beneficial to subjects (such as by reducing or preventing infection).

The immune reactions, also known as immune responses, refer to actions of cells of an immune system (such as T lymphocytes, B lymphocytes, natural killer (NK) cells, macrophages, eosinophils, mast cells, dendritic cells, and neutrophils) and soluble macromolecules (including antibodies, cytokines, and complements) produced by any one of these cells or by the liver, leading to selective targeting, binding, damaging, destruction, and/or exclusion of invading pathogens, cells or tissues infected with the pathogens, cancer cells, or other abnormal cells in vertebrates, or normal human cells or tissues in a case of autoimmune or pathological inflammations. When being administrated to the subjects, the composition contains a certain amount of the polypeptide or the antigenic fragment thereof of the present disclosure, sufficient to elicit the immune reactions. The composition used as a vaccine includes the polypeptide or the antigenic fragment thereof of the present disclosure, and any other component required. An "immunologically effective amount" means that the amount is administrated to the subject by a single dose or as a series of portions, being effective to treatment or prevention.

In some embodiments, the composition of the present disclosure further comprises one or more adjuvants. The "adjuvant" usually refers to a reagent that increases, stimulates, activates, enhances or regulates the immune reactions against active ingredients of the composition at the cellular level or body fluid level. The adjuvant can play a role in both acquired immunity and innate immunity, and exert functions in a variety of ways.

Many substances (natural or synthetic) have been shown to work as the adjuvants. Known adjuvants described in the literature may be used, for example, Clin Microbiol Rev 1994, 7: 277-89. Examples of suitable adjuvants include, but not limited to: aluminum salts (aluminum phosphate, aluminum hydroxide, hydroxyl aluminum oxide, etc.), alum, cholera toxin, Salmonella toxin, an incomplete Freund's adjuvant (IFA), a complete Freund's adjuvant (CFA), ISCOMatrix, GM-CSF, other immunostimulating cytokines, oligodeoxynucleotides containing CpG motifs (CpG7909, etc.), oil-in-water emulsions, saponins or derivatives thereof (QS21, etc.), lipopolysaccharides such as lipid A or derivatives thereof (MPL, RC529, GLA, E6020, etc.), lipopeptides, lactoferrin, a flagellin protein, double-stranded RNA or derivatives thereof (poli IC, etc.), bacterial DNA, imidazoquinoline (Imiquimod, R848, etc.), C-type lectin ligands (trehalose-6,6'-dibehenate (TDB), etc.), CD1d ligands (alpha-galactosylceramide, etc.), squalene emulsions (MF59, AS03, AF03, etc.), PLGA, etc. The adjuvants and the composition may be administered continuously to the subject or mixed together before they are to be administered to the subject.

In another aspect, the present disclosure provides a composition containing the antibodies described herein further contains a carrier and/or an excipient.

The carrier includes any and all pharmaceutically acceptable solvents, dispersion media, vehicles, coatings, diluents, antibacterial agents and antifungal agents, isotonic agents and delayed absorbents, buffers, carrier solutions, suspensions, colloids, etc. The use of such media and reagents for pharmaceutically active substances is well known in the art. Supplemented active ingredients may also be introduced into the composition.

The term "pharmaceutically acceptable" refers to that a molecule or a composition, when being administrated to a recipient, is harmless to the recipient or has benefits to the recipient over any harmful effect. For the carrier or the excipient for preparing the composition disclosed herein, a pharmaceutically acceptable carrier or excipient is necessary to be compatible with other components of the composition, and is harmless to its recipient, or has benefits to its recipient over any harmful effect. The term "pharmaceutically acceptable carrier" refers to pharmaceutically acceptable materials, compositions or vehicles, such as liquid or solid fillers, diluents, excipients, solvents, media, encapsulation materials, manufacturing aids (such as lubricants, magnesium talc, calcium stearate or zinc or stearic acid), or solvent encapsulation materials, which are involved in carrying or transporting reagents from one portion of the body to another portion (for example, from one organ to another organ). Each carrier is necessary to be "acceptable" in the sense that it is compatible with other components of the formulation, and is not harmful to a patient. Some examples of materials that can serve as a pharmaceutically acceptable carrier include: (1) sugars, such as lactose, glucose, and sucrose; (2) starch, such as corn starch and potato starch; (3) cellulose and derivatives thereof, such as sodium carboxymethyl cellulose, methyl cellulose, ethyl cellulose, microcrystalline cellulose, and cellulose acetate; (4) powdery tragacanth gum; (5) malt; (6) gelatin; (7) excipients, such as cocoa butter and suppository wax; (8) oil, such as peanut oil, cottonseed oil, safflower oil, sesame oil, olive oil, corn oil, and soybean oil; (9) diols, such as propylene glycol; (10) polyols, such as glycerol, sorbitol, mannitol, and polyethylene glycol (PEG); (11) esters, such as ethyl oleate and ethyl laurate; (12) agar; (13) buffers, such as magnesium hydroxide and aluminum hydroxide; (14) alginic acid; (15) pyrogen-free water; (16) isotonic saline; (17) a Ringer's solution; (19) pH buffer solutions; (20) polyesters, polycarbonates, and/or polyanhydrides; (21) fillers, such as polypeptides and amino acids; (22) serum components, such as serum albumin, HDL, and LDL; (23) C2-C12 alcohols, such as ethanol; and (24) other non-toxic compatible substances used in pharmaceutical preparations.

Suitable excipients will be compatible with the antibodies of the present disclosure. Well-known excipients in the art include, for example, fillers, adhesives, disintegrants, coating agents, adsorbents, anti-adhesion agents, flow aids, preservatives, antioxidants, flavoring agents, colorants, sweeteners, solvents, co-solvents, buffers, chelating agents, viscosity imparting agents, surfactants, diluents, wetting agents, carriers, the preservatives, emulsifiers, stabilizers and tension regulators. The excipients contained in the composition of the present disclosure depend on their administration ways, and the excipients suitable for the composition of the present disclosure are known in the art.

In some embodiments, the composition according to the present disclosure may be prepared into the following forms: an ointment, a gel, a paste, a liquid solution, a suspension, a tablet, a gelatin capsule, a capsule, a suppository, powder, nasal drops, or an aerosol, preferably into a form of an injection solution or the suspension. For example, for an injection, the composition is usually packaged into a form of a liquid suspension, which may be injected through a syringe or perfusion. In this respect, the composition is usually dissolved into a saline, physiological, isotonic or buffer solution that is compatible with a drug's use and known to those skilled in the art.

### Array

The present disclosure provides an array, including a solid substrate and a polypeptide immobilized on the solid substrate, where the polypeptide includes at least one polypeptide or antigenic fragment thereof of the present disclosure. In some embodiments, the polypeptide includes at least 2, at least 3, at least 5, at least 10, at least 15, at least 20, at least 25, at least 30, or at least 35 polypeptides or antigenic fragments thereof of the present disclosure, for example, includes 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35 polypeptides or antigenic fragments thereof of the present disclosure. In another embodiments, the polypeptide includes at least 2, at least 3, at least 4, at least 5, at least 6, at least 7, at least 8, or at least 9 polypeptides or antigenic fragments thereof of the present disclosure, for example, includes 2, 3, 4, 5, 6, 7, 8, or 9 polypeptides or antigenic fragments thereof of the present disclosure.

As well known in the art, the "array" is usually formed by a linear or two-dimensional structure with separate spacing (i.e., discrete) regions ("points"). Each array has a finite area, and is formed on a surface of the solid substrate. The array may further be of a bead structure, where each bead may be recognized by a molecular code or a color code, or may be recognized in a continuous fluid. Analysis may also be performed sequentially, in which a sample passes through a series of points, and each point adsorbs such molecules from the solution. The solid substrate used in the present disclosure is not specifically limited, as long as the polypeptide can be immobilized on the substrate. The solid substrate is usually glass or a polymer; and the most commonly used polymers are cellulose, polyacrylamide, nylon, polystyrene, polyvinyl chloride, or polypropylene. The solid substrate may be a tube, a bead, a disk, a silicon chip, a microwell plate, a polyvinylidene fluoride (PVDF) membrane, a nitrocellulose membrane, a nylon membrane, other porous membranes, a non-porous membrane (for example, plastics, a polymer, perspex, silicon, etc.), a large amount of polymer needles, or a large amount of microtitration pores, or any other surface form suitable for immobilization of the polypeptide, a polynucleotide and other suitable molecules and/or for an immunoassay. A binding method is well known in the art, and usually includes covalently binding by cross-linking or physical adsorbing the polypeptide, the polynucleotide, etc. to the solid substrate. A position of each point may be determined by using a well-known technology such as contact or non-contact printing, mask or lithography (Jenkins, R.E., Pennington, S.R. (2001, Proteomics, 2, 13-29), and Lalet al. (2002, Drug Discov Today 15; 7 (18 Suppl): S143-9)).

The array is usually a microarray. The "microarray" refers to an array having regions with a discrete region density of at least about 100/cm², preferably, at least about 1000/cm². The regions in the microarray have usual dimensions, for example, a diameter in a range of about 10-250 µm, and are separated from other regions in the array by roughly the same distance. The array may also be a macro array or a nano array. A method for making the array is well known by those skilled in the art.

In some embodiments, the array is a polypeptide chip. The "polypeptide chip" refers to a biochip suitable for capturing the polypeptide, which can be used for high-throughput screening. A surface of the polypeptide chip contains a plurality of addressable positions, each of which has a biologically specific component bound to it, such as the polypeptide or the antigenic fragment thereof.

It is well known that the IgG and IgM antibodies play an important role in being directed against viral infections such as infection with the SARS coronavirus and the MERS coronavirus. Conventional technologies used for studying patients' IgG and IgM responses include ELISA and the immune colloidal gold technique. These technologies usually test only a single target protein or antibody in a single reaction. In contrast, the polypeptide chip can measure proteome ranges of the antibody responses in a high-throughput format, thereby providing a more systematic description of these important antibody responses.

As disclosed herein, the polypeptide or the antigenic fragment thereof contained in the array may be from a polypeptide or an antigenic fragment thereof of the SARS-CoV-2 ORF1ab polyprotein, a polypeptide or an antigenic fragment thereof derived from the SARS-CoV-2 S protein, and/or a polypeptide or an antigenic fragment thereof derived from the SARS-CoV-2 N protein. In some embodiments, the polypeptide or the antigenic fragment thereof contained in the array may be from at least one of the polypeptide or the antigenic fragment thereof of the SARS-CoV-2 ORF1ab polyprotein, the polypeptide or the antigenic fragment thereof derived from the SARS-CoV-2 S protein, and/or the polypeptide or the antigenic fragment thereof derived from the SARS-CoV-2 N protein. For example, the polypeptide or the antigenic fragment thereof contained in the array may be from one of the polypeptide or the antigenic fragment thereof of the SARS-CoV-2 ORF1ab polyprotein, the polypeptide or the antigenic fragment thereof derived from the SARS-CoV-2 S protein, and/or the polypeptide or the antigenic fragment thereof derived from the SARS-CoV-2 N protein, or two of the polypeptide or the antigenic fragment thereof of the SARS-CoV-2 ORF1ab polyprotein, the polypeptide or the antigenic fragment thereof derived from the SARS-CoV-2 S protein, and/or the polypeptide or the antigenic fragment thereof derived from the SARS-CoV-2 N protein, or all the three of the polypeptide or the antigenic fragments thereof of the SARS-CoV-2 ORF1ab polyprotein, the polypeptide or the antigenic fragment thereof derived from the SARS-CoV-2 S protein, and/or the polypeptide or the antigenic fragment thereof derived from the SARS-CoV-2 N protein.

### Diagnosis and prognosis

The present disclosure provides a method for diagnosing whether a subject is infected with SARS-CoV-2 using the polypeptide or the antigenic fragment thereof of the present disclosure, or the array of the present disclosure. The method includes: detecting the presence of an antibody specifically bound to the polypeptide in a sample by contacting the sample of the subject with the polypeptide or the antigenic fragment thereof of the present disclosure, or the array of the present disclosure, thereby performing diagnosis or prognosis against the SARS-CoV-2 infection.

"Diagnosis" refers to identification on the presence or nature of a pathological condition, or a subtype of the pathological condition, that is, the presence or risk of COVID-19. "Prognosis" is used herein to refer to prediction on the likelihood of a disease or disease progression (including a relapse and a therapeutic reaction).

As used herein, the terms "subject", "individual", "host", and "patient" may be used interchangeably herein, and refer to any mammalian subject, especially a human, who is expected to undergo diagnose, prognosis or treatment. The method described herein is suitable for both human therapy and veterinary application. In some embodiments, the subject is a mammal; and in a specific embodiment, the subject is the human.

The "sample of the subject" refers to a biological sample from the subject that can be used for analysis, including a tissue or fluid sample taken from or derived from the individual. Suitable biological samples include, for example, whole blood, a serum, plasma, a blood portion, sputums, urine, a biopsy sample, a crude lysate, etc. The sample of a to-be-tested subject is preferably derived from the same species as a control sample.

### Induction of immune reaction

The present disclosure provides a method for inducing an immune response against SARS-CoV-2 in a subject using the polypeptide or the antigenic fragment thereof of the present disclosure, or the array of the present disclosure. The method includes: administering the polypeptide or the antigenic fragment thereof of the present disclosure, or the composition of the present disclosure (for example, a vaccine composition) to a subject, to expose the polypeptide or the antigenic fragment and/or the adjuvant thereof of the present disclosure to an immune system of the subject.

The term "induction of the immune reaction" refers to inducing an antigen-specific immune response at the first encounter of the immune system with at least one antigenic protein or antigenic fragment thereof and then within a limited period of time. The time period is, for example, at least 1 year, at least 2 years, at least 3 years, at least 5 years, or at least 10 years, before induction. In one embodiment, the encounter of an individual or subject's immune system with the antigenic protein or the antigenic fragment thereof without inducing the antigen-specific immune response is not considered as "induction of the immune reaction". For example, the encounter of the individual or subject's immune system with the antigenic protein or the antigenic fragment thereof without inducing a persistent immunity is not considered as "induction of the immune reaction" according to the present disclosure. In yet another embodiment, induction of the persistent immunity is mediated by production of memory B cells and/or memory T cells. In a case of a cancer, for example, a specific antigen may be expressed by cancer cells without eliciting the immune reaction. Only the presence of the antigen is not "induction of the immune reaction" against the antigen as understood in the present application. In one embodiment, the individual or the subject has not intentionally immunized with the antigenic protein or the antigenic fragment thereof, or the vector containing a nucleic acid encoding such protein or fragment, to treat or prevent a disease within the given time period as previously mentioned.

Administration may occur once or for many times. In one embodiment, one or more administrations may occur as a portion of the so-called "prime and boost" solution. Other administration systems may include delayed release or sustained release delivery systems.

The composition of the present invention may be stored in a solution or freeze-dried form. Preferably, solutions are freeze-dried in the presence of sugars such as the sucrose or the lactose. It is still preferred that they are freeze-dried, and reconstituted before use.

The composition may be presented in a form of a cartridge containing the composition and the adjuvant or a reconstitution solution; and the reconstitution solution contains one or more pharmaceutically acceptable diluents to facilitate reconstitution of the composition administrated to the mammal using conventional or other devices. This cartridge may optionally include a device (for example, a hypodermic syringe and a microneedle array) for administrating a liquid form of the composition and/or instructions.

The composition disclosed herein may be combined into various delivery systems. For example, in some embodiments, the composition may be applied to a "microneedle array" or "microneedle patch" delivery system for administration.

### Prevention and treatment

The present disclosure provides a method for preventing and/or treating a disease caused by SARS-CoV-2 infection in a subject. The method includes: administering the polypeptide or the antigenic fragment thereof of the present disclosure, the composition of the present disclosure, or the antibody of the present disclosure to the subject.

As used herein, "prevention" means administrating a therapeutically effective amount of the polypeptide or the antigenic fragment thereof, the composition, or the antibody of the present disclosure to the subject, so as to protect the subject from a disease caused by SARS-CoV-2 infection. When the term "prevention" used herein is related to a given treatment on a given condition (for example, prevention of SARS-CoV-2 infection), it is intended to express that a treated subject do not develop a clinically observable condition level at all, or develop more slowly and/or to a lesser degree than a subject not treated. These terms are not limited to a situation in which the subject does not experience any condition. For example, if treatment is given upon a patient exposed to a stimulus that is expected to generate manifestation of the given condition, and the treatment causes the subject to experience fewer and/or milder symptoms of the condition than expected without giving the treatment, then the treatment may be considered to have prevented the condition. When the subject is brought to present only mild symptoms of obvious infection, treatment is considered to "prevent" infection, which does not mean that no cell is necessary to be penetrated by the virus.

As used herein, "treatment" refers to a method of obtaining beneficial or desirable results (including clinical results). For the purposes of the present invention, beneficial or desirable clinical results include but not limited to one or more of the following: alleviating one or more symptoms caused by the disease, reducing the degree of the disease, stabilizing the disease (for example, preventing or delaying disease deterioration), preventing or delaying spread of the disease, preventing or delaying the disease relapse, delaying or slowing the disease progression, improving a disease state, providing disease remission (partially or completely), reducing a dose of one or more other drugs required to treat the disease, delaying the disease progression, increasing or improving the quality of life, increasing a body weight, and/or prolonging a survival time. The method of the present invention envisages any one or more of these aspects of treatment.

In some embodiments, treatment may be given after one or more symptoms have already appeared. In other embodiments, treatment may be given without the symptoms. For example, treatment may be given to a susceptible individual before the onset of the symptoms, or may be performed with another damaging agent (for example, according to a history of the symptoms, and according to genetic or other susceptibility factors, disease therapies, or any combination of them). Treatment may also be continued after the symptoms have regressed, for example, to prevent or delay their relapse.

Similarly, reduction (for example, reduction in the risk of SARS-CoV-2 infection) used herein in connection to the infection risk for a given treatment refers to slower or milder development of infection in the subject compared to a basic development level of infection in control or absence of treatment (for example, administration of the polypeptide of the present disclosure). Reduction in the infection risk may cause the subject to present only mild symptoms of obvious infection or delayed symptoms of infection, which does not mean that no cell is necessary to be penetrated by the virus.

As used herein, "administration" refers to introduction of the composition or a reagent into the subject, including introduction of the composition or the reagent in parallel or in sequence. "Administration" may refer to, for example, treatment, pharmacokinetics, diagnosis, a research, a placebo, and an experimental method. "Administration" also covers in vitro and in vitro treatment. The composition or the reagent is introduced into the subject by any suitable route. Examples of suitable methods for administrating the polypeptide, the antibody, or the composition of the present disclosure include, but not limited to, oral, intradermal, subcutaneous, intramuscular, intraosseous, intraperitoneal, and intravenous injections, as well as systemic or topical administration at a site proximate a target site. Administration includes self-administration and administration by other persons. Administration may be performed in any suitable route. A suitable administration route allows the composition or the reagent to perform its desired functions. For example, if the suitable route is intravenous, the composition is administered by introducing the composition or the reagent into a vein of the subject.

In some embodiments of the present disclosure, the polypeptide, the antibody, or the composition of the present disclosure is packaged together with or stored in an apparatus for administration. Apparatuses for injectable preparations include, but not limited to, injection ports, automatic syringes, injection pumps, and injection pens. Apparatuses for nebulized preparations or powder preparations include, but not limited to, inhalers, insufflators, aspirators, etc. Therefore, the present disclosure includes an administration apparatus containing the polypeptide, the antibody, or the composition of the present disclosure for treating or preventing one or more of disorders described herein.

The "subjects" intended to be administrated include, but not limited to, the humans (i.e., males or females in any age group, for example, child subjects (for example, infants, children, adolescents), or adult subjects (for example, young, middle-aged, or older persons)) and/or other non-human animals, for example, mammals (for example, primates).

### Examples

The following further describes the present disclosure in combination with specific embodiments; and the advantages and the features of the present disclosure will be obvious with the description. However, these examples are only exemplary, and should not be construed as limiting the present disclosure. Those skilled in the art should appreciate that modifications and substitutions could be made on details and forms without departing from the spirit and scope of the present disclosure, but all fall within the scope of protection of the present disclosure.

### Example 1. Induction of persistent neutralizing antibodies in SARS-CoV-2 infection

First, serum samples of SARS-CoV-2 infected patients were tested for overall neutralizing antibodies. Titers of an antigen-specific IgG antibody against a SARS-CoV-2 N or S protein in the serum samples at different time points after the onset of symptoms were measure with ELISA (on Days 1-30, n = 36; on Days 31-61, n = 18; on Days 100-150, n = 21; and on Days 180-220, n = 26), where a control group was serum samples of 20 healthy donors (HDs). Results showed that compared with the healthy donors, significantly more IgG antibodies against the N protein and the S protein were produced in the patients with COVID-19 within 30 days after the onset of the symptoms (Fig. 1A and Fig. 1B).

In addition to the determination of the binding antibodies, a pseudotype virus was further used to determine a dynamic response of a functional neutralizing antibody in an individual with COVID-19. More than 94% (34/36) of the serum samples, collected from 4 days to 30 days after the onset of the symptoms, of 36 patients showed strong antibody neutralizing activity against a SARS-CoV-2 pseudovirus. This result showed that a specific antibody neutralization response is produced in each patient infected with SARS-CoV-2.

### Example 2. Synthesis of polypeptides derived from the novel coronavirus and manufacture of polypeptide chips

According to an amino acid sequence of a SARS-CoV-2 Wuhan-Hu-1 strain, a total of 515 polypeptides covering an entire SARS-CoV-2 proteome were designed, where each polypeptide was 15 amino acids in length, overlapping with an adjacent polypeptide by 11 amino acids. The polypeptides were synthesized by GL Biochem (Shanghai, China) Ltd. Sulfo-SMCC (ThermoFisher, MA, USA) was used to couple the polypeptides to BSA. Briefly, BSA was activated with Sulfo-SMCC at a molar ratio of 1: 30, and then dialyzed in a PBS buffer. Cysteine residues were added to the C-termini of the polypeptides, to couple to BSA using their sulfhydryl groups. Cysteine-contained peptides were added at a ratio of 1: 1 (w/w) for incubation for 2 h, and then dialyzed with PBS to remove free peptides.

Three replicates of each synthetic polypeptide and negative (BSA) and positive controls (anti-human IgG (Cat # 12136) and IgM (Cat # 12386) antibodies) were printed on a PATH backsheet (GraceBio-Labs, USA), and chips were generated using a Super Marathon printer (Arrayjet, UK). The polypeptide chips were then stored at -80°C until use.

### Example 3. Serum antibody detection and data analysis

The polypeptide chips were heated to a room temperature, and then incubated in blocking buffers (a PBS buffer containing 3% BSA and a PBS buffer containing 0.1% Tween20) for 3 h. The serum samples from the patients with COVID-19 or combined serum samples from 20 healthy donors (a control group) were diluted in PBS containing 0.1% Tween20, followed by incubation with the polypeptide chips at 4°C for 2 h. The polypeptide chips were washed with 1x PBST, and then incubated with cy3-conguated goat-anti-human IgG and AlexaFluor647-conjugated donkey-anti-human IgM (Jackson Immuno Research, Pennsylvania, USA, Cat # 109-165-008 and Cat # 709-605-073) diluted with 1x PBST at 1: 1000 at the room temperature for 1 h. After incubation, the polypeptide chips were washed with 1x PBST, and then centrifuged and dried at the room temperature. Then, the chips were scanned with LuxScan10K-A (Beijing CapitalBio, China). The parameters were set to 95% laser power/PMT550 and 95% laser power/PMT480 respectively. Fluorescence intensity data was obtained by GenePixPro6.0 software (Molecular Devices, CA, USA). Fig. 2 was a schematic picture showing obtained fluorescence intensity data, where green fluorescence indicated an IgG signal intensity, red fluorescence indicated an IgM signal intensity, and yellow was superposition of green and red.

Data about IgG and IgM are analyzed separately. For each point, the signal intensity was defined as a foreground minus a background, and an average of three points of each polypeptide was taken. A critical value of a positive peptide reaction of each COVID-19 individual sample was set to twice the signal intensity of the healthy donor control group. Peptides with positive rates exceeding 80% at all three sampling time points (10-60 days, 100-150 days, 180-220 days after onset) were defined as "dominant and persistent epitopes"; and peptides with positive rates exceeding 60% at the three time points were defined as "subdominant and persistent epitopes". An average signal intensity of each peptide in the three sampling time point groups was calculated, and the peptides with higher signal intensities (i.e., higher than the average signal intensity plus a standard deviation) were selected. R software (version 3.6.3) was used for data processing and analysis, and a change on significant signal intensity between different sampling time points groups was evaluated based on the Limma algorithm. P value less than 0.05 indicated that a difference was statistically significant.

### Example 4. Identification on dominant antigen epitopes

A total of 51 longitudinal serum samples from 19 patients with mild to moderate (n = 17), asymptomatic (n = 1) and severe (n = 1) SARS-CoV-2 infection were detected using the polypeptide chips. Neutralizing antibodies were induced and maintained in all the patients except one patient with asymptomatic infection (data not shown). Serum samples were collected at 2 or 3 time points from 16 days to 219 days after the onset of symptoms in each patient with COVID-19 in order. According to different sampling time points, the samples were divided into three groups: Days 10-60 (n = 18), Days 100-150 (n = 18), and Days 180-220 (n = 15). Viral epitope profiles of the serum IgG and IgM antibodies in individuals with COVID-19 were evaluated longitudinally using the combined serum of the 20 healthy donors as a negative control.

The response kinetics of peptide-specific antibodies was determined by the SARS-CoV-2 polypeptide chips; and the binding signal intensity and a percentage of the response positive samples for each peptide (i.e., the positive rate) were analyzed. Based on the critical value (set to twice the signal intensity of the negative control) of the positive peptide binding response, a total of 460 IgG positive peptides and 479 IgM positive peptides were identified in the condition that at least one patient's serum sample is necessary to have the positive response. The results showed that response distribution of SARS-CoV-2 in different ORF proteins was relatively stable over time, where the replicase polyprotein ORF1ab had the most positive responses (Fig.3).

On the basis of the identified positive epitopes, epitopes to which a strong antibody response was always maintained in more than 80% of the samples in the three sampling groups were selected, called the dominant and persistent epitopes. The results showed that these highly dominant epitopes capable of mediating long-term humoral immune reactions were located in the ORF1ab polyprotein and the S protein of SARS-CoV-2; and the epitopes recognized by the IgM antibody (n = 33) were more than those recognized by the IgG antibody (n = 10) (Table 1, Fig. 4). Among them, the ORF1ab polyprotein had the most dominant epitopes, and its epitopes were widely distributed in regions of non-structural proteins (nsp) 2-5, nsp8-10, nsp12-14, and nsp16.

Noted that one immunodominant epitope No. 2073 (SEQ ID NO.7, ORF1ab, aa 5801-5815) was identified; and it could be recognized by the IgG and IgM antibodies in 100% of sera of individuals with COVID-19 regardless of the serum sampling time point (Fig. 4). This highly reactive peptide was located in a helicase (nsp13) region of the ORF1ab polyprotein, essential for unlocking a double-stranded RNA template during SARS-CoV-2 replication (Chen, J. et al. Structural Basis for Helicase-Polymerase Coupling in the SARS-CoV-2 Replication-Transcription Complex. Cell 182, 1560-1573 e1513, doi: 10.1016/j. cell. 2020. 07. 033 (2020)).

A fold change on signal intensity of each peptide relative to the healthy control group was further calculated. Among the ORF1ab peptides, immunodominant peptides with the highest average signal intensity recognized by the two IgG antibodies-No.1985 (SEQ ID NO. 6, ORF1ab, aa 5449-5463) and No.2073 (SEQ ID NO. 7, ORF1ab, aa 5801-5815) were both located on nsp13. For IgM reaction, No. 685 (SEQ ID NO. 11, ORF1ab, aa 249-263) on nsp2 and No. 1985 (SEQ ID NO. 6, ORF1ab, aa 5449-5463) on nsp13 had the strongest binding intensity (Fig. 4).

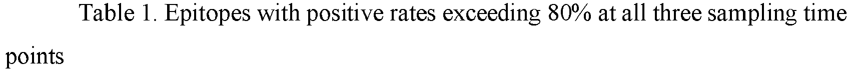

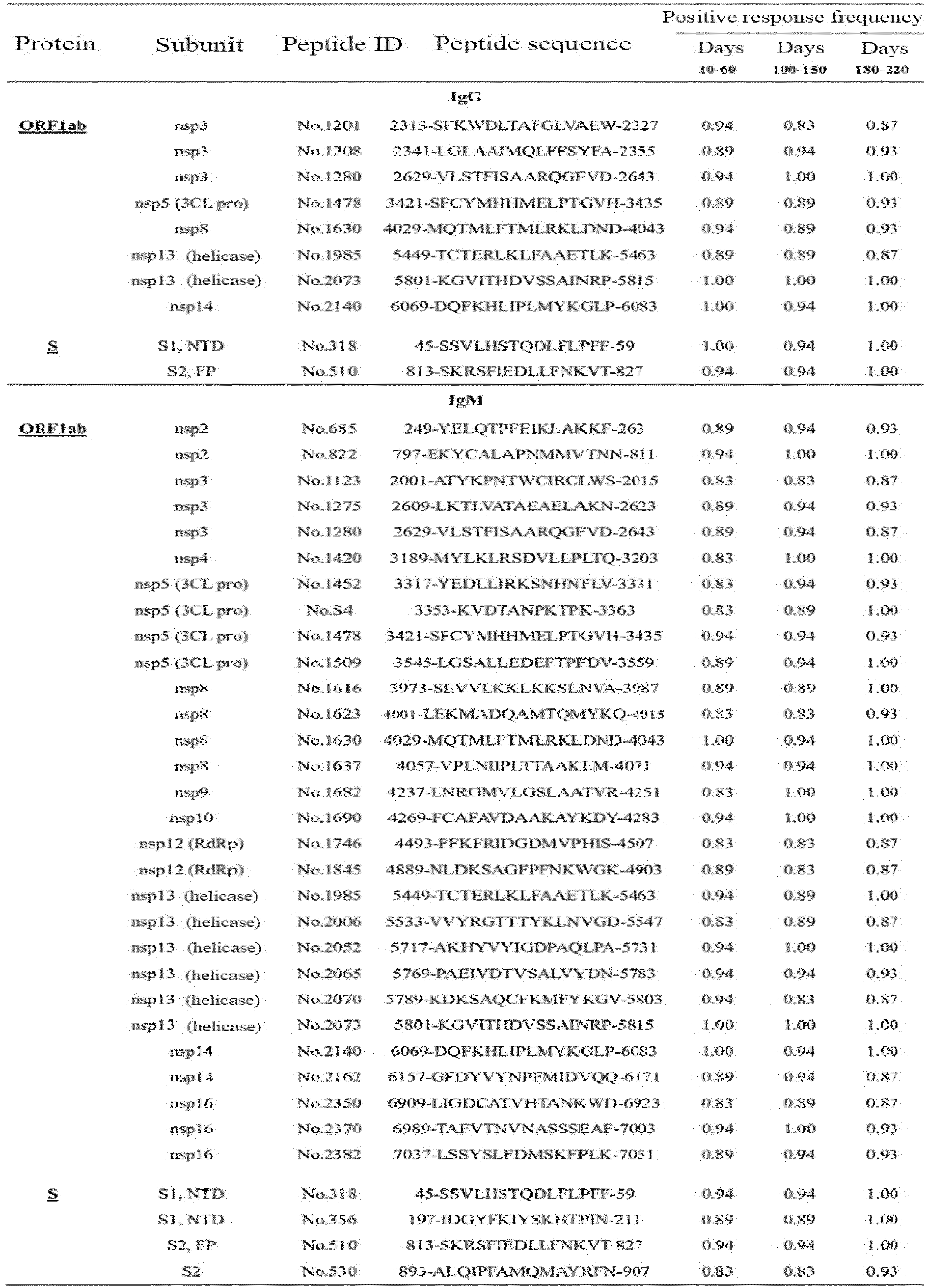

A total of 4 dominant and persistent epitopes were identified from the S protein of SARS-CoV-2: peptides with numbers No. 318 (SEQ ID NO. 9, S, aa 45-59) and No. 356 (SEQ ID NO. 34, S, aa 197-211), located in the NTD; No. 510 (SEQ ID NO. 10, S, aa 813-827), covering a cleavage site of S2 and fusion peptide (FP) regions of some S2 subunits; and No. 530 (SEQ ID NO. 35, S, aa 893-907), located in a linking region between the FP and the first heptad repeat (HR1) of the S2 subunit. Among these peptides, the peptide No. 318 located on the NTD of the S protein had the strongest binding intensity.

Structural analysis showed that in an S protein monomer, these epitopes were all exposed to a surface of the S protein monomer. However, in the trimeric S protein, the polypeptides No. 318, No. 356, and No. 530 were hidden inside the trimeric protein (Fig. 5). The results that these peptides were identified as the dominant and persistent epitopes showed that both the S protein monomer and the trimeric structure could be effectively recognized by a host immune system in vivo.

Considering that new SARS-CoV-2 variations emerge worldwide, a sequence alignment was further performed on the dominant epitopes No. 318 (SEQ ID NO. 9), No. 356 (SEQ ID NO. 34), No. 510 (SEQ ID NO. 10), and No. 530 (SEQ ID NO. 35) in the S protein of early SARS-CoV-2 (Wuhan-Hu-1 strain) and 6 variant strains (B.1.1.7, B.1.351, B.1.429, P.1, B.1.525, and B.1.617). The results showed that sequences of these dominant and persistent epitopes were identical in different virus strains except for a Q52R variation on No. 318 in the B.1.525 virus strain (Fig. 6). It indicated that these epitopes were highly conservative, and potentially mediated a persistent long-term antibody response after infection with SARS-CoV-2 variants.

### Example 5. Identification on subdominant and persistent epitopes

The dominant and persistent epitopes defined above were not found on the N protein (the positive rates at all the three sampling time points were more than 80%). Subsequently, a second round of epitope screening was performed according to the data obtained by the polypeptide chips, to select the subdominant and persistent epitopes to which the positive responses were always maintained in more than 60% of the COVID-19 samples at the three sampling time points.

A total of 4 subdominant and persistent epitopes of the N protein were identified (Table 2, Fig. 7). Among them, peptide No. 2455 (SEQ ID NO. 36, N, aa 213-227) had a reaction to both the IgG and IgM antibodies of the patients with COVID-19, having a higher signal intensity; two overlapping peptides No. 2455 (SEQ ID NO. 36, N, aa 213-227) and No. 2456 (SEQ ID NO. 37, N, aa 217-231) were located in the Ser/Arg-rich linking region between the N-terminal RNA binding domain and the C-terminal dimerization domain of the N protein; and polypeptides No. 2482 (SEQ ID NO. 38, N, aa 321-335) and No. 2491 (SEQ ID NO. 39, N, aa 357-371) were completely or partially located in the dimerization domain of the N protein.

**Table 2. Epitopes with positive rates exceeding 60% at all three sampling time points**

| Protein | | Subunit | Peptide ID | Peptide sequence | Positive response frequency | | |
|---|---|---|---|---|---|---|---|
| | | | | | Days **10-60** | Days **100-150** | Days **180-210** |
| | linker rich in Ser/Arg | | | **IgG** | | | |
| **N** | | | No.2455 | 213-GGDAALALLLLDRL-227 | 0.83 | 0.83 | 0.80 |
| | | | | **IgM** | | | |
| **N** | linker rich in Ser/Arg | | No.2455 | 213-GGDAALALLLLDRL-227 | 0.72 | 0.61 | 0.67 |
| linker rich in Ser/Arg | | | No.2456 | 217-AALALLLLDRLNQLE-231 | 0.78 | 0.78 | 0.80 |
| Dimerization domain | | | No.2482 | 321-GMEVTPSGTWLTYTG-335 | 0.72 | 0.78 | 0.87 |
| Dimerization domain/C-terminal domain | | | No.2491 | 357-IDAYKTFPPTEPKKD-371 | 0.67 | 0.67 | 0.67 |

Structural analysis on the epitopes No. 2482 and No. 2491 located in the C-terminal dimerization domain of the N protein was performed. An amino acid sequence of the peptide No. 2482 formed two β chains, which are arranged in a dimer in an antiparallel manner; while the amino acid sequence at 357-364 of the peptide No. 2491 formed a helical structure at two opposite ends of the dimer (Fig. 8).

### Example 6. Identification of epitopes with high signal intensities and reduced reactivity over time

In addition to identification of the above highly reactive epitopes responsible for persistent humoral immune reaction, a group of 9 positive peptides (Table 3) were further identified, showing strong binding intensity (i.e., higher than an average of the signal intensity of all the test samples plus SD), but the reactivity of each serum sample was decreased over time (the positive rate changed by more than 20%). Such change trend was consistent with an overall trend of the decreased humoral immune reactions in the patients. These 9 epitopes were located within the three dominant reactive antigens S, N and ORF1ab proteins (Fig. 9).

**Table 3. Epitopes with high signal intensities and reduced reactivity over time**

| Protein | Subunit | Peptide ID | Peptide sequence | Positive response frequency | | |
|---|---|---|---|---|---|---|
| | | | | Days **10-60** | Days **100-150** | Days **180-220** |
| | | | **IgG** | | | |
| **ORF1ab** | nsp2 | No.784 | 645-GWEIVKFISTCACEI-659 | 0.61 | 0.28 | 0.40 |
| | nsp3 | No.1040 | 1669-WADNNCYLATALLTL-1683 | 0.61 | 0.50 | 0.33 |
| | nsp10 | No.1693 | 4281-KDYLASGGQPITNCV-4295 | 0.67 | 0.39 | 0.40 |
| | nsp14 | No.2212 | 6357-DKSAFVNLKQLPFFY-6371 | 0.78 | 0.61 | 0.33 |
| **S** | S2, FP | No.511 | 817-FIEDLLFNKVTLADA-831 | 0.78 | 0.61 | 0.47 |
| **N** | linker rich in Ser/Arg | No.2457 | 221-LLLLDRLNQLESKMS-235 | 0.61 | 0.39 | 0.33 |
| | | | **IgM** | | | |
| **ORF1ab** | nsp8 | No.1617 | 3977-LKKLKKSLNVAKSEF-3991 | 0.61 | 0.28 | 0.40 |
| | nsp13 (helicase) | No.1986 | 5453-RLKLFAAETLKATEE-5467 | 0.72 | 0.39 | 0.47 |
| | nsp14 | No.2159 | 6145-ASDTYACWHHSIGFD-6159 | 0.89 | 0.83 | 0.67 |

Among these 9 epitopes, the signal intensities of three peptides (No. 784, SEQ ID NO. 40-IgG, No. 1617, SEQ ID NO. 46-IgM, No. 1986, SEQ ID NO. 47-IgM) in ORF1ab and one peptide (No. 2457, SEQ ID NO. 45-IgG) in the N protein were significantly decreased between samples at the early sampling time (10-60 days after onset) and the samples at the late sampling time (100-150 days or 180-220 days after onset) (p < 0.05) (Fig. 10). Based on this feature, the humoral immune reactions to these peptides could be used as indicators to monitor disease progression after SARS-CoV-2 infection and/or to study prognosis of the patients.

### Example 7. Construction of novel coronavirus infection diagnosis model based on dominant and persistent epitopes

A total of 75 human serum samples were collected, and the antibody responses of the polypeptides in the samples were detected using the novel coronavirus polypeptide chips made in Example 2. The 75 samples included 24 samples from healthy donors uninfected with the novel coronavirus, 18 samples from patients recovered for 1 month after novel coronavirus infection, 18 samples from patients recovered for 4 months after novel coronavirus infection, and 15 samples from patient recovered for 7 months after novel coronavirus infection.

Antibody response intensities in each sample of 35 dominant and persistent epitope peptides (Table 1) identified in Example 4 were extracted from the detection results of the polypeptide chips, to explore whether there was a subset (panel) in the 35 peptides with higher antibody responses in the patients infected with the novel coronavirus, which could accurately distinguish between or predict the samples infected and uninfected with the novel coronavirus. A novel coronavirus diagnosis model was constructed based on this subset.

Specifically, the serum samples of the patients infected with the novel coronavirus were used as case groups based on January, April, and July respectively, combined with the uninfected samples (as a control group) in order and analyzed. First, correlation in antibody response intensities between any two of the 35 polypeptides was analyzed, and data about one of the two peptides with close correlation was removed. Subsequently, feature screening was performed to explore a predictor capable of accurately predicting the patients infected with the novel coronavirus using some polypeptides. The predictor used randomForest, lasso, elasticNet, svmLinear and svmRadial algorithms for calculation. A panel-based score of each sample was calculated according to an optimal polypeptide subset produced by each algorithm. A ROC curve was drawn using this score, and an AUC score (the closer the AUC is to 1, the higher the accuracy is), specificity, sensitivity, and a size of the polypeptides contained in the subset were used as a standard for evaluating a model obtained by each algorithm.

### Results:

### 1. Results obtained according to IgM response intensity:

The performance of the polypeptide subset screened by different algorithms and the prediction model constructed based on the IgM response intensity of these polypeptides was summarized as shown in the following table:

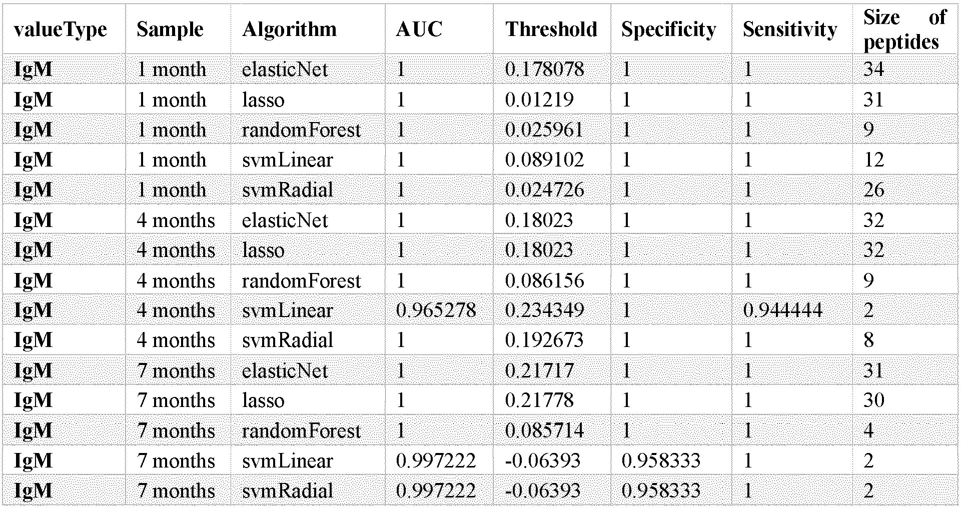

Among them, the peptide size of the subset obtained by the randomForest algorithm was moderate, and the model prediction accuracy was high. The models of the samples infected with the novel coronavirus in January, April and July were predicted based on IgM by using the randomForest algorithm was shown in the following table:

SampleAUC Threshold Specificity Sensitivity Peptide number (No.) Size of peptides

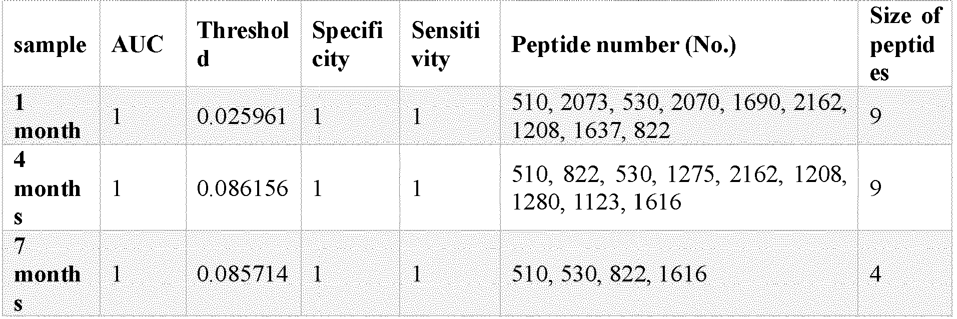

### 2. Results obtained according to IgG response intensity:

The performance of the polypeptide subset screened by different algorithms and the prediction model constructed based on the IgG response intensity of these polypeptides was summarized as shown in the following table:

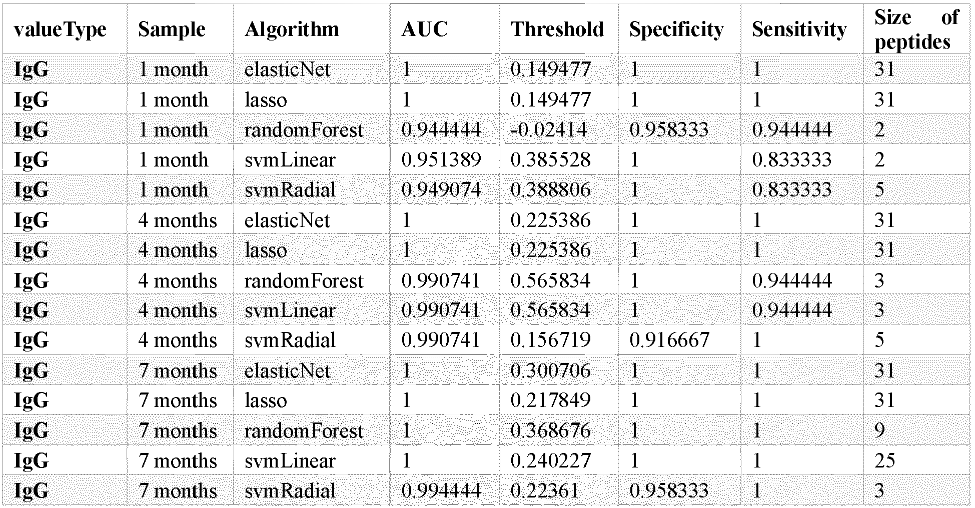

Among them, the peptide size of the subset obtained by the randomForest algorithm was moderate, and the model prediction accuracy was high. The models of the samples infected with the novel coronavirus in January, April and July were predicted based on IgG by using the randomForest algorithm was shown in the following table:

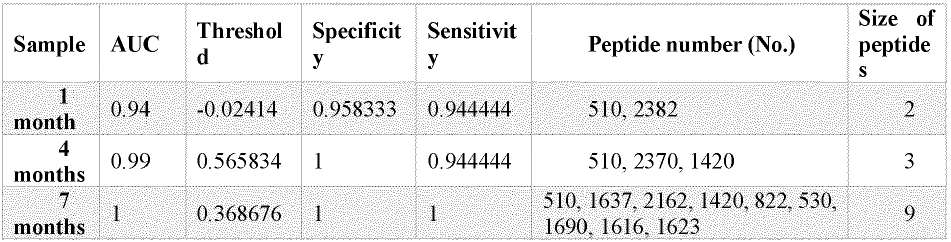

## Claims

1. An isolated polypeptide or an antigenic fragment thereof, wherein the polypeptide has an amino acid sequence selected from a group consisting of the following amino acid sequences or amino acid sequences obtained after a substitution, a deletion, an insertion and/or an addition of one or more amino acid residues has been made in the following amino acid sequences:
SFKWDLTAFGLVAEW (SEQ ID NO: 1);
LGLAAIMQLFFSYFA (SEQ ID NO: 2);
VLSTFISAARQGFVD (SEQ ID NO: 3);
SFCYMHHMELPTGVH (SEQ ID NO: 4);
MQTMLFTMLRKLDND (SEQ ID NO: 5);
TCTERLKLFAAETLK (SEQ ID NO: 6);
KGVITHDVSSAINRP (SEQ ID NO: 7);
DQFKHLIPLMYKGLP (SEQ ID NO: 8);
SSVLHSTQDLFLPFF (SEQ ID NO: 9);
SKRSFIEDLLFNKVT (SEQ ID NO: 10);
YELQTPFEIKLAKKF (SEQ ID NO: 11);
EKYCALAPNMMVTNN (SEQ ID NO: 12);
ATYKPNTWCIRCLWS (SEQ ID NO: 13);
LKTLVATAEAELAKN (SEQ ID NO: 14);
MYLKLRSDVLLPLTQ (SEQ ID NO: 15);
YEDLLIRKSNHNFLV (SEQ ID NO: 16);
KVDTANPKTPK (SEQ ID NO: 17);
LGSALLEDEFTPFDV (SEQ ID NO: 18);
SEWLKKLKKSLNVA (SEQ ID NO: 19);
LEKMADQAMTQMYKQ (SEQ ID NO: 20);
VPLNIIPLTTAAKLM (SEQ ID NO: 21);
LNRGMVLGSLAATVR (SEQ ID NO: 22);
FCAFAVDAAKAYKDY (SEQ ID NO: 23);
FFKFRIDGDMVPHIS (SEQ ID NO: 24);
NLDKSAGFPFNKWGK (SEQ ID NO: 25);
VVYRGTTTYKLNVGD (SEQ ID NO: 26);
AKHYVYIGDPAQLPA (SEQ ID NO: 27);
PAEIVDTVSALVYDN (SEQ ID NO: 28);
KDKSAQCFKMFYKGV (SEQ ID NO: 29);
GFDYVYNPFMIDVQQ (SEQ ID NO: 30);
LIGDCATVHTANKWD (SEQ ID NO: 31);
TAFVTNVNASSSEAF (SEQ ID NO: 32);
LSSYSLFDMSKFPLK (SEQ ID NO: 33);
IDGYFKIYSKHTPIN (SEQ ID NO: 34);
ALQIPFAMQMAYRFN (SEQ ID NO: 35);
GGDAALALLLLDRL (SEQ ID NO: 36);
AALALLLLDRLNQLE (SEQ ID NO: 37);
GMEVTPSGTWLTYTG (SEQ ID NO: 38); and
IDAYKTFPPTEPKKD (SEQ ID NO: 39).

2. An isolated polypeptide or an antigenic fragment thereof, wherein the polypeptide has an amino acid sequence selected from a group consisting of the following amino acid sequences or amino acid sequences obtained after a substitution, a deletion, an insertion and/or an addition of one or more amino acid residues has been made in the following amino acid sequences:
SFKWDLTAFGLVAEW (SEQ ID NO: 1);
LGLAAIMQLFFSYFA (SEQ ID NO: 2);
VLSTFISAARQGFVD (SEQ ID NO: 3);
SFCYMHHMELPTGVH (SEQ ID NO: 4);
MQTMLFTMLRKLDND (SEQ ID NO: 5);
TCTERLKLFAAETLK (SEQ ID NO: 6);
KGVITHDVSSAINRP (SEQ ID NO: 7);
DQFKHLIPLMYKGLP (SEQ ID NO: 8);
SSVLHSTQDLFLPFF (SEQ ID NO: 9);
SKRSFIEDLLFNKVT (SEQ ID NO: 10);
YELQTPFEIKLAKKF (SEQ ID NO: 11);
EKYCALAPNMMVTNN (SEQ ID NO: 12);
ATYKPNTWCIRCLWS (SEQ ID NO: 13);
LKTLVATAEAELAKN (SEQ ID NO: 14);
MYLKLRSDVLLPLTQ (SEQ ID NO: 15);
YEDLLIRKSNHNFLV (SEQ ID NO: 16);
KVDTANPKTPK (SEQ ID NO: 17);
LGSALLEDEFTPFDV (SEQ ID NO: 18);
SEWLKKLKKSLNVA (SEQ ID NO: 19);
LEKMADQAMTQMYKQ (SEQ ID NO: 20);
VPLNIIPLTTAAKLM (SEQ ID NO: 21);
LNRGMVLGSLAATVR (SEQ ID NO: 22);
FCAFAVDAAKAYKDY (SEQ ID NO: 23);
FFKFRIDGDMVPHIS (SEQ ID NO: 24);
NLDKSAGFPFNKWGK (SEQ ID NO: 25);
WYRGTTTYKLNVGD (SEQ ID NO: 26);
AKHYVYIGDPAQLPA (SEQ ID NO: 27);
PAEIVDTVSALVYDN (SEQ ID NO: 28);
KDKSAQCFKMFYKGV (SEQ ID NO: 29);
GFDYVYNPFMIDVQQ (SEQ ID NO: 30);
LIGDCATVHTANKWD (SEQ ID NO: 31);
TAFVTNVNASSSEAF (SEQ ID NO: 32);
LSSYSLFDMSKFPLK (SEQ ID NO: 33);
IDGYFKIYSKHTPIN (SEQ ID NO: 34); and
ALQIPFAMQMAYRFN (SEQ ID NO: 35).

3. The polypeptide or the antigenic fragment thereof according to claim 1 or 2, wherein the antigenic fragment is at least 5 amino acids, at least 6 amino acids, at least 7 amino acids, at least 8 amino acids, at least 9 amino acids, or at least 10 amino acids in length.

4. An isolated polypeptide or an antigenic fragment thereof, wherein the polypeptide has an amino acid sequence selected from a group consisting of the following amino acid sequences or amino acid sequences obtained after a substitution, a deletion, an insertion and/or an addition of one or more amino acid residues has been made in the following amino acid sequences:
GWEIVKFISTCACEI (SEQ ID NO: 40);
WADNNCYLATALLTL (SEQ ID NO: 41);
KDYLASGGQPITNCV (SEQ ID NO: 42);
DKSAFVNLKQLPFFY (SEQ ID NO: 43);
FIEDLLFNKVTLADA (SEQ ID NO: 44);
LLLLDRLNQLESKMS (SEQ ID NO: 45);
LKKLKKSLNVAKSEF (SEQ ID NO: 46);
RLKLFAAETLKATEE (SEQ ID NO: 47); and
ASDTYACWHHSIGFD (SEQ ID NO: 48).

5. The polypeptide or the antigenic fragment thereof according to claim 4, wherein the antigenic fragment is at least 5 amino acids, at least 6 amino acids, at least 7 amino acids, at least 8 amino acids, at least 9 amino acids, or at least 10 amino acids in length.

6. An isolated antibody, binding to the polypeptide or the antigenic fragment thereof according to any one of claims 1-5.

7. The antibody according to claim 6, wherein the antibody is selected from an IgG antibody, an IgM antibody and an IgA antibody.

8. The antibody according to claim 7, wherein the antibody is selected from IgG1, IgG2, IgG3 and IgG4 antibodies.

9. The antibody according to any one of claims 6-8, wherein the antibody is a monoclonal antibody.

10. The antibody according to any one of claims 6-9, wherein the antibody is a human antibody.

11. A composition, comprising one or more polypeptides or antigenic fragments thereof according to any one of claims 1-5.

12. The composition according to claim 11, wherein the composition comprises a polypeptide or an antigenic fragment thereof having an amino acid sequence shown as SEQ ID NO: 10 and one or more of polypeptides or antigenic fragments thereof having an amino acid sequence selected from amino acid sequences shown as SEQ ID NOs: 1-9 and 11-48.

13. The composition according to claim 11 or 12, wherein the composition comprises the polypeptides or the antigenic fragments thereof having the amino acid sequences shown as SEQ ID NOs: 10, 7, 35, 29, 23, 30, 2, 21, and 12.

14. The composition according to claim 11 or 12, wherein the composition comprises the polypeptides or the antigenic fragments thereof having the amino acid sequences shown as SEQ ID NOs: 10, 12, 35, 14, 30, 2, 3, 13, and 19.

15. The composition according to claim 11 or 12, wherein the composition comprises the polypeptides or the antigenic fragments thereof having the amino acid sequences shown as SEQ ID NOs: 10,35, 12, and 19.

16. The composition according to claim 11 or 12, wherein the composition comprises the polypeptides or the antigenic fragments thereof having the amino acid sequences shown as SEQ ID NOs: 10 and 33.

17. The composition according to claim 11 or 12, wherein the composition comprises the polypeptides or the antigenic fragments thereof having the amino acid sequences shown as SEQ ID NOs: 10, 32, and 15.

18. The composition according to claim 11 or 12, wherein the composition comprises the polypeptides or the antigenic fragments thereof having the amino acid sequences shown as SEQ ID NOs: 10, 21, 30, 15, 12, 35, 23, 19, and 20.

19. The composition according to any one of claims 11-18, wherein the antigenic fragment is at least 5 amino acids, at least 6 amino acids, at least 7 amino acids, at least 8 amino acids, at least 9 amino acids, or at least 10 amino acids in length.

20. The composition according to any one of claims 11-19, wherein the composition is a vaccine composition, and optionally, further comprises one or more adjuvants.

21. A composition, comprising one or more antibodies according to any one of claims 6-10, and an optional carrier and/or excipient.

22. A nucleotide sequence, encoding the polypeptide or the antigenic fragment thereof according to any one of claims 1-5, or the antibody according to any one of claims 6-10.

23. A vector, comprising the nucleotide sequence according to claim 22.

24. An array, comprising a solid substrate and a polypeptide immobilized on the solid substrate, wherein the polypeptide comprises at least one polypeptide or antigenic fragment thereof according to any one of claims 1-3.

25. The array according to claim 24, wherein the polypeptide comprises at least 2, at least 3, at least 5, at least 10, at least 15, at least 20, at least 25, at least 30, or at least 35 polypeptides or antigenic fragments thereof according to any one of claims 1-3.

26. The array according to claim 24 or 25, wherein the polypeptide comprises a polypeptide or an antigenic fragment thereof having an amino acid sequence shown as SEQ ID NO: 10, and one or more of polypeptides or antigenic fragments thereof having amino acid sequences selected from amino acid sequences shown as SEQ ID NOs: 1-9 and 11-48.

27. The array according to any one of claims 24-26, wherein the polypeptide comprises the polypeptides or the antigenic fragments thereof having the amino acid sequences shown as SEQ ID NOs: 10, 7, 35, 29, 23, 30, 2, 21, and 12.

28. The array according to any one of claims 24-26, wherein the polypeptide comprises the polypeptides or the antigenic fragments thereof having the amino acid sequences shown as SEQ ID NOs: 10, 12, 35, 14, 30, 2, 3, 13, and 19.

29. The array according to any one of claims 24-26, wherein the polypeptide comprises the polypeptides or the antigenic fragments thereof having the amino acid sequences shown as SEQ ID NOs: 10, 35, 12, and 19.

30. The array according to any one of claims 24-26, wherein the polypeptide comprises the polypeptides or the antigenic fragments thereof having the amino acid sequences shown as SEQ ID NOs: 10 and 33.

31. The array according to any one of claims 24-26, wherein the polypeptide comprises the polypeptides or the antigenic fragments thereof having the amino acid sequences shown as SEQ ID NOs: 10, 32, and 15.

32. The array according to any one of claims 24-26, wherein the polypeptide comprises the polypeptides or the antigenic fragments thereof having the amino acid sequences shown as SEQ ID NOs: 10, 21, 30, 15, 12, 35, 23, 19, and 20.

33. The array according to any one of claims 24-32, wherein the antigenic fragment is at least 5 amino acids, at least 6 amino acids, at least 7 amino acids, at least 8 amino acids, at least 9 amino acids, or at least 10 amino acids in length.

34. The array according to claim 24, wherein the polypeptide comprises the polypeptide or the antigenic fragment thereof according to any one of claims 1-3, wherein the plurality of polypeptides or antigenic fragments thereof comprise at least one polypeptide or antigenic fragment thereof derived from a SARS-CoV-2 ORF1ab polyprotein, and at least one polypeptide or antigenic fragment thereof derived from a SARS-CoV-2 S protein.

35. The array according to claim 34, wherein the plurality of polypeptides further comprise at least one polypeptide or antigenic fragment thereof derived from a SARS-CoV-2 N protein.

36. An array, comprising a solid substrate and a polypeptide immobilized on the solid substrate, wherein the polypeptide comprises at least one polypeptide or antigenic fragment thereof according to claim 4 or 5.

37. The array according to claim 36, wherein the polypeptide comprises at least 2, at least 3, at least 4, at least 5, at least 6, at least 7, at least 8, or all 9 polypeptides or antigenic fragments thereof according to claim 4 or 5.

38. The array according to claim 36, wherein the polypeptide comprises a plurality of polypeptides or antigenic fragments thereof according to claim 4 or 5, wherein the plurality of polypeptides or antigenic fragments thereof comprise at least one polypeptide or antigenic fragment thereof derived from a SARS-CoV-2 ORF1ab polyprotein, at least one polypeptide or antigenic fragment thereof derived from a SARS-CoV-2 S protein, and at least one polypeptide or antigenic fragment thereof derived from a SARS-CoV-2 N protein.

39. The array according to any one of claims 24-38, wherein the polypeptide is immobilized on the solid substrate after being coupled to BSA.

40. The array according to any one of claims 24-39, wherein the array is a polypeptide chip.

41. A method for detecting the presence of an antibody against SARS-CoV-2 in a subject, comprising
(a) contacting a sample from the subject with the polypeptide or the antigenic fragment thereof according to any one of claims 1-3, or the array according to any one of claims 24-40; and
(b) detecting the presence of the antibody in the sample specifically bound to the polypeptide or the polypeptide on the array.

42. The method according to claim 41, wherein the method is used for diagnosing whether the subject is infected with SARS-CoV-2.

43. A method for the prognosis of a subject infected with SARS-CoV-2, comprising
(a) contacting a sample from the subject with the polypeptide or the antigenic fragment thereof according to claim 4 or 5, or the array according to any one of claims 36-40; and
(b) detecting the presence of an antibody in the sample specifically bound to the polypeptide or the polypeptide on the array.

44. The method according to any one of claims 41-43, wherein the antibody is an IgG antibody or an IgM antibody.

45. The method according to any one of claims 41-44, wherein the sample is a blood sample, for example, a serum or plasma.

46. A method for inducing an immune response against SARS-CoV-2 in a subject, comprising administrating the polypeptide or the antigenic fragment thereof according to any one of claims 1-5, or the composition according to any one of claims 11-20 to the subject.

47. A method for preventing and/or treating a disease caused by SARS-CoV-2 infection in a subject, comprising administrating the polypeptide or the antigenic fragment thereof according to any one of claims 1-5, or the composition according to any one of claims 11-20 to the subject.

48. The method according to claim 46 or 47, comprising administrating at least 2, at least 3, at least 5, at least 10, at least 15, at least 20, at least 25, or at least 30 polypeptides or antigenic fragments thereof according to any one of claims 1-5 to the subject.

49. The method according to claim 46 or 47, comprising the step of: administrating the polypeptide or the antigenic fragment thereof according to any one of claims 1-5 to the subject, wherein the plurality of polypeptides or antigenic fragments thereof comprise at least one polypeptide or antigenic fragment thereof derived from a SARS-CoV-2 ORF1ab polyprotein, at least one polypeptide or antigenic fragment thereof derived from a SARS-CoV-2 S protein, and at least one polypeptide or antigenic fragment thereof derived from a SARS-CoV-2 N protein.

50. A method for treating a disease caused by SARS-CoV-2 infection in a subject, comprising administrating the antibody according to any one of claims 6-10 to the subject.
